# EUROPÄISCHE PATENTSCHRIFT

(11) **EP 2 665 463 B1**
(45) Veröffentlichungstag und Bekanntmachung des Hinweises auf die Patenterteilung: **28.06.2017**
(21) Anmeldenummer: 12700409.1
(22) Anmeldetag: 19.01.2012
(51) Int. Cl.: A61K 8/86, A61K 8/58, A61K 8/92, A61Q 15/00, A61K 8/02

(54) **ANTITRANSPIRANT-STIFTE MIT VERBESSERTER LANGZEITSTABILITÄT**
ANTIPERSPIRANT STICK WITH IMPROVED LONG-TERM STABILITY
STICK ANTITRANSPIRANT AYANT UNE MEILLEURE STABILITÉ À LONG TERME

(30) Priorität: 19.01.2011 DE 102011002863
(43) Veröffentlichungstag der Anmeldung: 27.11.2013
(73) Patentinhaber: Henkel AG & Co. KGaA, 40589 Düsseldorf (DE)
(72) Erfinder: ANDERHEGGEN, Bernd, 41189 Mönchengladbach (DE); CLAAS, Marcus, 40723 Hilden (DE); BANOWSKI, Bernhard, 40597 Düsseldorf (DE)
(86) Internationale Anmeldenummer: PCT/EP2012/050789
(87) Internationale Veröffentlichungsnummer: WO 2012/098189

(56) Entgegenhaltungen:
- DE-A1-102005 029 776
- DE-A1-102005 029 777
- DE-A1-102006 004 957

## Beschreibung

Die vorliegende Anmeldung beschäftigt sich mit wasserfreien Antitranspirant-Stiften auf Wachsbasis, die eine verbesserte Langzeitstabilität aufweisen.

Wasserfreie Antitranspirant-Zusammensetzungen, die als Stift auf Wachsbasis konfektioniert sind, enthalten neben teilchenförmigen schweißreduzierenden Wirkstoffen in der Regel mindestens ein kosmetisches Öl als Träger für den teilchenförmigen schweißreduzierenden Wirkstoff. Damit sich der im Öl suspendierte Antitranspirantwirkstoff bei der Lagerung nicht absetzt, enthalten handelsübliche Stifte Verdickungsmittel, wie Fettalkohole und/oder Wachse.

Bei den handelsüblichen Antitranspirant-Stiften ist der in dem wasserfreien, ölhaltigen Träger suspendierte Antitranspirantwirkstoff mit einer Ölschicht bedeckt. Diese Ölschicht verzögert allerdings die Freisetzung des Antitranspirantwirkstoffs in die wirksame wasserlösliche Form. Um dies zu vermeiden, enthalten wasserfreie Antitranspirant-Stifte auf Wachsbasis üblicherweise einen Öl-in-Wasser-Emulgator, der auf der Haut unter dem Einfluss der Hautfeuchtigkeit und des Schweißes die Freisetzung des Antitranspirantwirkstoffs aus der Wachsmatrix begünstigt.

Der vorliegenden Anmeldung lag die Beobachtung zu Grunde, dass bei solchen Stiften des Standes der Technik die Interaktion zwischen Antitranspirantwirkstoff, Öl-in-Wasser-Emulgator und Luftfeuchtigkeit (bei längerer Lagerung) bzw. Hautfeuchtigkeit oder Schweiß (durch Hautkontakt bei der Applikation) negative Veränderungen der Stiftoberfläche hervorruft. Die Stiftoberfläche wird hart, die Produktabgabe beim Bestreichen der Haut verringert sich und die kosmetischen Eigenschaften des Stiftes verschlechtern sich. Die schweißhemmende Wirkung des Produkts lässt bereits nach 2 bis 3 Wochen regelmäßiger Gebrauchszeit merklich nach.

Aufgabe der vorliegenden Erfindung war es daher, wasserfreie Antitranspirant-Stifte auf Wachsbasis bereitzustellen, die auch nach vielen Gebrauchszyklen unter Feuchtigkeitseinfluss (Luft/ Haut/Schweiß) keine Verschlechterung der Abgabeeigenschaften zeigen, wobei unter Abgabeeigenschaften insbesondere das Anschmelzverhalten und Gleitvermögen des Stiftes beim Auftragen auf die Haut, aber auch die Freisetzung des Antitranspirantwirkstoffs und damit die Antitranspirantleistung, zu verstehen ist.

Überraschend wurde nun gefunden, dass bei wasserfreien Antitranspirant-Stiften auf Wachsbasis die vorstehend genannten Nachteile beseitigt oder zumindest deutlich vermindert und die Langzeitstabilität der Abgabeeigenschaften und die Resistenz gegenüber Feuchtigkeitseinfluss deutlich verbessert werden kann, wenn die Stifte, jeweils bezogen auf ihr Gesamtgewicht, 1 - 4 Gew.-%, bevorzugt 2 - 3,5 Gew.-%, besonders bevorzugt 2,5 - 3 Gew.-%, mindestens eines Öl-in-Wasser-Emulgators, ausgewählt aus Ethoxylaten von C₁₀-C₂₂-Alkanolen mit 8 bis 16, bevorzugt 10 - 14, besonders bevorzugt mit 12 Ethylenoxid-Einheiten im Molekül enthalten.

Gegenstand der vorliegenden Anmeldung sind Antitranspirant-Wachsstifte, enthaltend insgesamt 3 - 27 Gew.-%, bezogen auf das Gesamtgewicht der kristallwasserfreien Aktivsubstanz (USP) in der Gesamtzusammensetzung, Antitranspirant-Wirkstoff(e),
0 bis maximal 4 Gew.-% freies Wasser,
insgesamt 30 - 70 Gew.-% an mindestens einem unter Normalbedingungen flüssigen kosmetischen Öl, darin 10 - 50 Gew.-% mindestens eines flüchtigen Öls, das ausgewählt ist aus flüchtigen cyclischen Siliconen, flüchtigen linearen Siliconen und flüchtigen aliphatischen Kohlenwasserstoffen sowie Mischungen hiervon, und zusätzlich zu dem mindestens einen flüchtigen Öl insgesamt 1 - 30 Gew.-% mindestens eines nicht-flüchtigen Öls,
insgesamt 15 - 25 Gew.-% mindestens einer unter Normalbedingungen festen Wachskomponente,
darin 12 - 22 Gew.-% mindestens eines linearen und gesättigten C₁₄-C₂₂-Alkanols,
0,5 - 5 Gew.-% mindestens eines Wachses mit einem Schmelzpunkt von 65 - 150 °C,
0,5 - 8 Gew.-% mindestens eines Wachses mit einem Schmelzpunkt von 25 bis < 50°C,
0,5 - 6 Gew.-% mindestens eines wasserunlöslichen teilchenförmigen Füllstoffs, 1 - 4 Gew.-%, bevorzugt 2 - 3,5 Gew.-%, besonders bevorzugt 2,5 - 3 Gew.-%, mindestens eines Öl-in-Wasser-Emulgators, ausgewählt aus Ethoxylaten von C₁₀-C₂₂-Alkanolen mit 8 bis 16 Ethylenoxid-Einheiten im Molekül,
wobei sich, sofern nichts anderes vermerkt ist, alle Gew.-%-Angaben auf das Gesamtgewicht des Antitranspirant-Wachsstifts beziehen.

Gegenstand der vorliegenden Anmeldung sind auch Antitranspirant-Wachsstifte, enthaltend insgesamt 3 - 27 Gew.-%, bezogen auf das Gesamtgewicht der kristallwasserfreien Aktivsubstanz (USP) in der Gesamtzusammensetzung, Antitranspirant-Wirkstoff(e),
0 bis maximal 4 Gew.-% freies Wasser,
insgesamt 30 - 70 Gew.-% an mindestens einem unter Normalbedingungen flüssigen kosmetischen Öl, darin 10 - 50 Gew.-% mindestens eines flüchtigen Öls, das ausgewählt ist aus flüchtigen cyclischen Siliconen, flüchtigen linearen Siliconen und flüchtigen aliphatischen Kohlenwasserstoffen sowie Mischungen hiervon, und zusätzlich zu dem mindestens einen flüchtigen Öl insgesamt 1 - 30 Gew.-% mindestens eines nicht-flüchtigen Öls,
insgesamt 15 - 25 Gew.-% mindestens einer unter Normalbedingungen festen Wachskomponente, darin
insgesamt 12 - 22 Gew.-% mindestens eines linearen und gesättigten C₁₄-C₂₂-Alkanols, insgesamt 0,5 - 5 Gew.-% mindestens eines Wachses mit einem Schmelzpunkt von 65 - 150 °C, insgesamt 0,5 - 8 Gew.-% mindestens eines Wachses mit einem Schmelzpunkt von 25 bis < 50°C,
weiterhin insgesamt 0,5 - 6 Gew.-% mindestens eines wasserunlöslichen teilchenförmigen Füllstoffs,
insgesamt 1 - 4 Gew.-%, bevorzugt 2 - 3,5 Gew.-%, besonders bevorzugt 2,5 - 3 Gew.-%, mindestens eines Öl-in-Wasser-Emulgators, ausgewählt aus Ethoxylaten von C₁₀-C₂₂-Alkanolen mit 8 bis 16 Ethylenoxid-Einheiten im Molekül,
wobei sich, sofern nichts anderes vermerkt ist, alle Gew.-%-Angaben auf das Gesamtgewicht des Antitranspirant-Wachsstifts beziehen.

"Normalbedingungen" sind im Sinne der vorliegenden Anmeldung eine Temperatur von 20 °C und ein Druck von 1013 hPa. Schmelzpunktangaben beziehen sich ebenfalls auf einen Druck von 1013 hPa.

Der Gehalt der erfindungsgemäßen Stifte an freiem Wasser beträgt 0 - 4 Gew.-%, bevorzugt 0,5 - 3 Gew.-%, jeweils bezogen auf das Gesamtgewicht des Antitranspirant-Wachsstifts.

"Freies Wasser" im Sinne der vorliegenden Anmeldung ist Wasser, das nicht in Form von Kristallwasser, Hydratationswasser oder ähnlich molekular gebundenem Wasser in der Antitranspirant-Zusammensetzung enthalten ist. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist beispielsweise solches Wasser, das als Lösemittel, als Gelaktivator oder als Lösemittelbestandteil anderer Wirkstoffe zur erfindungsgemäßen Zusammensetzung zugegeben wird.

Bei den kosmetischen Ölen unterscheidet man flüchtige und nicht-flüchtige Öle. Unter nicht-flüchtigen Ölen versteht man erfindungsgemäß solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man erfindungsgemäß solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.

Die Begriffe "Antitranspirant-Wachsstift", "Antitranspirant-Stift", "Antitranspirant-Stift auf Wachsbasis", "Stiftzusammensetzung" und "erfindungsgemäße Zusammensetzung" werden mit Bezug auf den Gegenstand der vorliegenden Anmeldung synonym gebraucht.

Die erfindungsgemäßen Zusammensetzungen enthalten mindestens einen schweißhemmenden Wirkstoff, der auch als Antitranspirant-Wirkstoff bezeichnet wird, in einer Gesamtmenge von 3 - 27 Gew.-%, bevorzugt 5 - 22 Gew.-% und besonders bevorzugt 10 - 20 Gew.-%, jeweils bezogen auf das Gesamtgewicht der kristallwasserfreien Aktivsubstanz (USP) in der Gesamtzusammensetzung.

Bevorzugte Antitranspirant-Wirkstoffe sind ausgewählt aus den wasserlöslichen adstringierenden anorganischen und organischen Salzen des Aluminiums, Zirconiums und Zinks bzw. beliebigen Mischungen dieser Salze.

Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Die schweißhemmenden Wirkstoffe liegen in ungelöster, suspendierter Form vor. Aus Gründen der Produktstabilität ist es bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglycol (PG) oder Aluminiumchlorhydrex-Polyethylenglycol (PEG), Aluminium-oder Aluminiumzirconium-Glycol-Komplexe, z. B. Aluminium- oder Aluminiumzirconium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat und Aluminiumzirconiumoctachlorhydrat sowie den Aluminium-Zirconium-Chlorohydrat-Glycin-Komplexen, wie Aluminiumzirconiumtrichlorhydrexglycin, Aluminiumzirconiumtetrachlorhydrexglycin, Aluminiumzirconiumpentachlorhydrexglycin und Aluminiumzirconiumoctachlorhydrexglycin, weiterhin aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirconiumchlorohydrat). Erfindungsgemäß besonders bevorzugte Antitranspirant-Wirkstoffe sind ausgewählt aus Aluminiumchlorhydrat und Aluminiumzirconiumchlorhydraten, insbesondere aus Aluminium-Zirconium-Tetrachlorohydrex-Glycin-Komplexen.

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium-und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(AI+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(AI+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(AI+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der l-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze, wie sie beispielsweise in US 6902723 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(AI+Zr)-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt. Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein AI:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein AI:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen.

Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind. Diese Zirconiumsalze sind beispielsweise in der belgischen Schrift BE 825146 offenbart.

Weitere bevorzugte schweißhemmende Wirkstoffe sind in US 6663854 und US 20040009133 offenbart.

Bevorzugte schweißhemmende Aluminium-Zirconium-Salze weisen ein molares Metall-zu-Chlorid-Verhältnis von 0,9 - 1,5, bevorzugt 0,9 - 1,3, besonders bevorzugt 0,9 - 1,1, auf. Erfindungsgemäß besonders bevorzugte Zirconium-freie Aluminiumsalze weisen ein molares Metall-zu-Chlorid-Verhältnis von 1,9 - 2,1 auf. Erfindungsgemäß besonders bevorzugte Zirconium-freie Aluminiumsesquichlorohydrate weisen ein molares Metall-zu-Chlorid-Verhältnis von 1,5:1-1,8:1 auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (AI+Zr)-zu-Chlorid-Verhältnis von 1,3 - 0,9), bevorzugt m = 0,91 - 1,11 (entsprechend M:CI = 1,1 - 0,9), und besonders bevorzugt m = 1,00 - 1,11 (entsprechend M:CI = 1,0 - 0,9), weiterhin sehr bevorzugt m = 1,02 - 1,11 (entsprechend M:CI = 0,98 - 0,9) sowie sehr bevorzugt m = 1,04 - 1,11 (entsprechend M:CI = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrat-Glycin-Komplexe, insbesondere solche mit einem atomaren AI:Zr-Verhältnis von 2-6 und einem molaren Metall-zu-Chlorid-Verhältnis M:CI von 0,9 - 1,5, bevorzugt Aluminium-Zirconiumtetrachlorohydrat-Glycin-Komplexe mit einem molaren Metall-zu-Chlorid-Verhältnis von 1,0 - 1,4, besonders bevorzugt 1,1 - 1,3.

Weiterhin erfindungsgemäß bevorzugt sind Aluminiumzirconiumchlorohydrat-Glycin-Komplexe, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf. Entsprechende Verbindungen sind beispielsweise offenbart in US 7105691.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze mit einem hohen HPLC-Peak 5-Aluminium-Gehalt (auch als "E⁵AZCH" bezeichnet) sind beispielsweise offenbart in US 6436381 und US 6649152.

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt.

Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind beispielsweise in US 6923952 offenbart.

In einer besonders bevorzugten Ausführungsform enthält die Zusammensetzung ein adstringierendes Aluminiumsalz, insbesondere Aluminiumchlorhydrat, das beispielsweise pulverförmig als Micro Dry^{®} Ultrafine oder Superultrafine von Reheis, Microdry 323 oder Micro Dry 3115 von Summit, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Besonders bevorzugt sind auch aktivierte Aluminiumchlorhydrate, die unter den Bezeichnungen Reach^{®} 101 und Reach^{®} 103, AACH- 7171 von Summit von Summit erhältlich sind. Auch die Verwendung von Aluminium-Zirconium-Tetrachlorohydrex-Glycin-Komplexen, die beispielsweise von Reheis unter der Bezeichnung Rezal^{®} 36 GP von Reheis oder AZG-364 oder 369 von Summit, in aktivierter Qualität, als Reach^{®} AZP-908, als Pulver im Handel sind, kann erfindungsgemäß besonders bevorzugt sein. Auch Aluminium-Zirconiumpentachlorohydex-Glycin-Komplexe (AAZG-3108 oder AAZG-3110 von Summit) sind bevorzugte Antitranspirant-Wirkstoffe.

Erfindungsgemäß besonders bevorzugte Antitranspirant-Stifte enthalten ein adstringierendes Aluminiumsalz mit einer zahlenmittleren Partikelgröße von 1 - 50 µm, bevorzugt 3 - 20 µm und besonders bevorzugt 5 - 10 µm, ausgewählt aus Aluminiumchlorhydrat, Aluminiumsesquichlorohydrat, Aluminium-Zirconium-Tetrachlorohydrex-Glycin-Komplexen Aluminium-Zirconiumpentachlorohydex-Glycin-Komplexen und Aluminium-Zirconiumoctachlorohydex-Glycin-Komplexen sowie Mischungen hiervon.

Die erfindungsgemäßen Antitranspirant-Stifte enthalten weiterhin insgesamt 30 - 70 Gew.-% an mindestens einem unter Normalbedingungen flüssigen kosmetischen Öl, darin 10 - 50 Gew.-% mindestens eines flüchtigen Öls, das ausgewählt ist aus flüchtigen cyclischen Siliconen, flüchtigen linearen Siliconen und flüchtigen aliphatischen Kohlenwasserstoffen sowie Mischungen hiervon, und zusätzlich zu dem mindestens einen flüchtigen Öl insgesamt 1 - 30 Gew.-% mindestens eines nicht-flüchtigen Öls enthalten sind, wobei sich alle Gew.-%-Angaben auf das Gesamtgewicht des Antitranspirant-Wachsstifts beziehen.

Derartige Mischungen aus flüchtigen und nicht-flüchtigen Ölen sorgen für ein angenehmes, nichtfettendes Hautgefühl und eine gute Wirkstofffreisetzung einerseits und andererseits für eine gute Maskierung sichtbarer Rückstände, die durch die pulverförmigen Stiftbestandteile entstehen können.

Die Gesamtmenge an unter Normalbedingungen flüssigen kosmetischen Ölen beträgt 30 - 70 Gew.-%, bevorzugt 39 - 60 Gew.-%, besonders bevorzugt 42 - 54 Gew.-%, außerordentlich bevorzugt 45 - 49 Gew.-%, jeweils bezogen auf das Gesamtgewicht des Antitranspirant-Wachsstifts.

### Flüchtige Öle

Bevorzugte erfindungsgemäße Antitranspirant-Wachsstift enthalten das mindestens eine flüchtige Öl, das ausgewählt ist aus flüchtigen cyclischen Siliconen, flüchtigen linearen Siliconen und flüchtigen aliphatischen Kohlenwasserstoffen sowie Mischungen hiervon, in einer Gesamtmenge von 15 - 40 Gew.-%, besonders bevorzugt 20 - 35 Gew.-%, außerordentlich bevorzugt 25 - 30 Gew.-%, jeweils bezogen auf das Gesamtgewicht des Stifts.

Bevorzugte flüchtige cyclische Silicone sind solche mit der INCI-Bezeichnung Cyclomethicone, insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan), sowie Mischungen hiervon. Diese Öle weisen einen Dampfdruck bei 20°C von ca. 13 - 15 Pa auf. Bevorzugte Antitranspirant-Stifte enthalten, jeweils bezogen auf das Gesamtgewicht des Antitranspirant-Wachsstifts, 10 - 50 Gew.-%, bevorzugt 15 - 40 Gew.-%, besonders bevorzugt 20 - 35 Gew.-%, außerordentlich bevorzugt 25 - 30 Gew.-% Cyclomethicone. Weitere bevorzugte Antitranspirant-Stifte enthalten, jeweils bezogen auf das Gesamtgewicht des Antitranspirant-Wachsstifts, 10 - 50 Gew.-%, bevorzugt 15 - 40 Gew.-%, besonders bevorzugt 20 - 35 Gew.-%, außerordentlich bevorzugt 25 - 30 Gew.-% Cyclopentasiloxan. Weitere bevorzugte Antitranspirant-Stifte enthalten, jeweils bezogen auf das Gesamtgewicht des Antitranspirant-Wachsstifts, 10 - 50 Gew.-%, bevorzugt 15 - 40 Gew.-%, besonders bevorzugt 20 - 35 Gew.-%, außerordentlich bevorzugt 25 - 30 Gew.-% einer Cyclopentasiloxan-Cyclohexasiloxan-Mischung. Weitere bevorzugte erfindungsgemäße Antitranspirant-Wachsstift enthalten mindestens eine flüchtige Öl, das ausgewählt ist aus flüchtigen cyclischen Siliconen, flüchtigen linearen Siliconen und flüchtigen aliphatischen Kohlenwasserstoffen sowie Mischungen hiervon, in einer Gesamtmenge von 15 - 40 Gew.-%, besonders bevorzugt 20 - 35 Gew.-%, außerordentlich bevorzugt 25 - 30 Gew.-%, jeweils bezogen auf das Gesamtgewicht des Stifts, wobei das mindestens eine flüchtige Öl ausgewählt ist aus Cyclopentasiloxan.

Bevorzugte flüchtige lineare Siliconöle sind ausgewählt aus linearen Siliconölen mit 2 - 10, bevorzugt 3 - 6 Siloxaneinheiten, wie Hexamethyldisiloxan (L₂), Octamethyltrisiloxan (L₃), Decamethyltetrasiloxan (L₄), Dodecamethylpentasiloxan (L₅), wie sie z. B. in den Handelsprodukten DC 2-1184 (bzw. Xiameter 2-1184), Dow Corning^{®} 200 (0,65 cSt) bzw. Xiameter PMX 0.65 cSt und Dow Corning^{®} 200 (1,5 cSt) bzw. Xiameter PMX 1.5 cSt von Dow Corning enthalten sind, sowie aus niedermolekularem Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist. Bevorzugte Antitranspirant-Stifte enthalten, jeweils bezogen auf das Gesamtgewicht des Antitranspirant-Wachsstifts, 10 - 50 Gew.-%, bevorzugt 15 - 40 Gew.-%, besonders bevorzugt 20 - 35 Gew.-%, außerordentlich bevorzugt 25 - 30 Gew.-% mindestens eines linearen Siliconöls mit 2 - 10, bevorzugt 3 - 6 Siloxaneinheiten,. Weitere bevorzugte Antitranspirant-Stifte enthalten, jeweils bezogen auf das Gesamtgewicht des Antitranspirant-Wachsstifts, 10 - 50 Gew.-%, bevorzugt 15 - 40 Gew.-%, besonders bevorzugt 20 - 35 Gew.-%, außerordentlich bevorzugt 25 - 30 Gew.-% mindestens eines linearen Siliconöls, ausgewählt aus Octamethyltrisiloxan, Decamethyltetrasiloxan und Dodecamethylpentasiloxan sowie Mischungen hiervon.

Bevorzugte flüchtige aliphatische Kohlenwasserstoffe sind ausgewählt aus C₈-C₁₆-Isoalkanen, insbesondere aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoalkan-Mischungen, insbesondere solche mit einem Dampfdruck bei 20°C von etwa 300 - 400 Pa, bevorzugt 360 Pa. Dieses mindestens eine C₈-C₁₆-Isoalkan ist bevorzugt in einer Gesamtmenge von 10 - 50 Gew.-%, bevorzugt 15 - 40 Gew.-%, besonders bevorzugt 20 - 35 Gew.-%, außerordentlich bevorzugt 25 - 30 Gew.-%, jeweils bezogen auf den gesamten Antitranspirant-Stift, enthalten. Weitere bevorzugte Antitranspirant-Stifte enthalten, jeweils bezogen auf das Gesamtgewicht des Stiftes, 10 - 50 Gew.-%, bevorzugt 15 - 40 Gew.-%, besonders bevorzugt 20 - 35 Gew.-%, außerordentlich bevorzugt 25 - 30 Gew.-% mindestens eines C₈-C₁₆-Isoalkans, ausgewählt aus Isodecan, Isoundecan, Isododecan und Isotridecan sowie Mischungen hiervon.

Weitere bevorzugte flüchtige aliphatische Kohlenwasserstoffe sind ausgewählt aus linearen C₉-C₁₇-Alkanen, insbesondere aus n-Nonan, n-Decan, n-Undecan, n-Dodecan, n-Tridecan, n-Tetradecan, n-Pentadecan, n-Hexadecan und n-Heptadecan, sowie Mischungen hiervon. Bevorzugte lineare C₉-C₁₇-Alkane sind ausgewählt aus n-Nonan, n-Undecan, n-Tridecan, n-Pentadecan und n-Heptadecan sowie Mischungen hiervon, insbesondere Mischungen aus n-Undecan und n-Tridecan. Bevorzugte Antitranspirant-Stifte enthalten, jeweils bezogen auf das Gesamtgewicht des Stiftes, 10 - 50 Gew.-%, bevorzugt 15 - 40 Gew.-%, besonders bevorzugt 20 - 35 Gew.-%, außerordentlich bevorzugt 25 - 30 Gew.-% mindestens eines linearen C₉-C₁₇-Alkans. Weitere bevorzugte Antitranspirant-Stifte enthalten, jeweils bezogen auf das Gesamtgewicht des Stiftes, 10 - 50 Gew.-%, bevorzugt 15 - 40 Gew.-%, besonders bevorzugt 20 - 35 Gew.-%, außerordentlich bevorzugt 25 - 30 Gew.-% n-Nonan, n-Undecan, n-Tridecan, n-Pentadecan und n-Heptadecan sowie Mischungen hiervon. Weitere bevorzugte Antitranspirant-Stifte enthalten, jeweils bezogen auf das Gesamtgewicht des Stiftes, 10 - 50 Gew.-%, bevorzugt 15 - 40 Gew.-%, besonders bevorzugt 20 - 35 Gew.-%, außerordentlich bevorzugt 25 - 30 Gew.-% einer Mischung aus n-Undecan und n-Tridecan.

### Nicht-flüchtige Öle

Die erfindungsgemäßen Antitranspirant-Stifte enthalten zusätzlich zu dem mindestens einen flüchtigen Öl mindestens ein nicht-flüchtiges Öl in einer Gesamtmenge von 1 - 30 Gew.-%, bevorzugt 5 - 25 Gew.-%, besonders bevorzugt 8 - 20 Gew.-%, außerordentlich bevorzugt 10 - 15 Gew.-%, wobei der Gesamtölgehalt 30 - 70 Gew.-% beträgt und sich alle Gew.-%-Angaben auf das Gesamtgewicht des Antitranspirant-Wachsstifts beziehen.

Erfindungsgemäß besonders bevorzugte nichtflüchtige Öle sind ausgewählt aus den Anlagerungsprodukten von 6 bis 20, bevorzugt 9 bis 15, Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (z. B. Ucon Fluid^{®} AP), PPG-9-Butylether (z. B. Breox^{®} B25), PPG-10-Butandiol und PPG-15-Stearylether. Besonders bevorzugt ist PPG-14-Butylether.

Erfindungsgemäß bevorzugte Antitranspirant-Stifte enthalten 1 - 30 Gew.-%, bevorzugt 5 - 25 Gew.-%, besonders bevorzugt 8 - 20 Gew.-%, außerordentlich bevorzugt 10 - 15 Gew.-%, mindestens eines Anlagerungsprodukts von 6 bis 20, bevorzugt 9 bis 15, Propylenoxid-Einheiten an ein ein- oder mehrwertiges C₃₋₂₂-Alkanol, wobei sich alle Gew.-%-Angaben auf das Gesamtgewicht des Antitranspirant-Wachsstifts beziehen. Weitere erfindungsgemäß bevorzugte Antitranspirant-Stifte enthalten 1 - 30 Gew.-%, bevorzugt 5 - 25 Gew.-%, besonders bevorzugt 8 - 20 Gew.-%, außerordentlich bevorzugt 10 - 15 Gew.-%, an PPG-14-Butylether oder PPG-15-Stearylether oder Mischungen aus PPG-14-Butylether und PPG-15-Stearylether, wobei sich alle Gew.-%-Angaben auf das Gesamtgewicht des Antitranspirant-Wachsstifts beziehen. Erfindungsgemäß besonders bevorzugte Antitranspirant-Stifte enthalten 8 - 20 Gew.-%, bevorzugt 10 - 15 Gew.-%, an PPG-14-Butylether, wobei sich alle Gew.-%-Angaben auf das Gesamtgewicht des Antitranspirant-Wachsstifts beziehen.

Weitere erfindungsgemäß besonders bevorzugte nichtflüchtige Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether.

Weitere erfindungsgemäß bevorzugte nicht-flüchtige Öle sind Ester der linearen oder verzweigten gesättigten oder ungesättigten Alkanole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Alkanole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isononylisononanoat, 2-Ethylhexylpalmitat und 2-Ethylhexylstearat. Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv^{®} TN (C₁₂-C₁₅-Alkylbenzoat), sowie Benzoesäureisostearylester, z. B. erhältlich als Finsolv^{®} SB, Ethylhexylbenzoat, z. B. erhältlich als Finsolv^{®} EB, und Benzoesäureoctyldocecylester, z. B. erhältlich als Finsolv^{®} BOD. Außerordentlich bevorzugt ist Benzoesäure-C₁₂-C₁₅-Alkylester.

Erfindungsgemäß bevorzugte Antitranspirant-Stifte enthalten, bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, mindestens eines Esters von linearen oder verzweigten gesättigten oder ungesättigten Alkanolen mit 2 - 30 Kohlenstoffatomen mit linearen oder verzweigten gesättigten oder ungesättigten Fettsäuren mit 2 - 30 Kohlenstoffatomen. Weitere erfindungsgemäß bevorzugte Antitranspirant-Stifte enthalten, bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, an mindestens einem Ester, ausgewählt aus Isopropylpalmitat, Isopropylstearat, Isopropylmyristat, 2-Propylheptyloctanoat (4-Methyl-2-pentylbutyloctansäureester), 2-Hexyldecylstearat, 2-Hexyldecyllaurat, Isononylisononanoat, 2-Ethylhexylpalmitat, 2-Ethylhexylstearat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat, Di-C₁₂-C₁₃-Alkylmalat, Benzoesäure-C₁₂-C₁₅-Alkylester (C₁₂-C₁₅-Alkylbenzoat), Benzoesäureisostearylester, 2-Ethylhexylbenzoat und Benzoesäureoctyldocecylester sowie Mischungen dieser Ester. Erfindungsgemäß besonders bevorzugte Antitranspirant-Stifte enthalten, bezogen auf das Gesamtgewicht des Stifts, 8 - 20 Gew.-%, bevorzugt 10 - 15 Gew.-%, an mindestens einem Ester, ausgewählt aus Isopropylpalmitat, Isopropylstearat, Isopropylmyristat, 2-Propylheptyloctanoat, 2-Ethylhexylpalmitat, 2-Ethylhexylstearat, Isononylisononanoat, Cetearylisononanoat, 2-Ethylhexyllaurat, 2-Ethylhexylisostearat, n-Hexyllaurat, Benzoesäure-C₁₂-C₁₅-Alkylester sowie Mischungen dieser Ester. Erfindungsgemäß besonders bevorzugt ist ein Gehalt an Benzoesäure-C₁₂-C₁₅-Alkylester von 8 - 20 Gew.-%, bevorzugt 10 - 15 Gew.-%, jeweils bezogen auf das Gesamtgewicht des Stifts.

Weitere erfindungsgemäß bevorzugte nichtflüchtige Öle sind ausgewählt aus verzweigten gesättigten oder ungesättigten Alkanolen mit 6 - 30 Kohlenstoffatomen. Diese Alkanole werden häufig auch als Guerbet-Alkohole bezeichnet, da sie nach der Guerbet-Reaktion erhältlich sind. Bevorzugte Guerbet-Alkohole sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z.B. 2-Hexyldecanol und 2-Hexyldecyllaurat.

Erfindungsgemäß bevorzugte Antitranspirant-Stifte enthalten, bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, mindestens eines verzweigten gesättigten oder ungesättigten Alkanols mit 6 - 30 Kohlenstoffatomen. Weitere erfindungsgemäß bevorzugte Antitranspirant-Stifte enthalten, bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, 2-Octyldodecanol.

Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol^{®} 318 oder Myritol^{®} 331 (BASF) mit unverzweigten Fettsäureresten sowie Glyceryltriisostearin und Glyceryltri(2-ethylhexanoat) mit verzweigten Fettsäureresten.

Erfindungsgemäß bevorzugte Antitranspirant-Stifte enthalten, bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, mindestens eines Triglycerids von linearen oder verzweigten, gesättigten oder ungesättigten, gegebenenfalls hydroxylierten C₈₋₃₀-Fettsäuren. Weitere erfindungsgemäß bevorzugte Antitranspirant-Stifte enthalten, bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, Capric/Caprylic Triglycerides.

Weitere erfindungsgemäß besonders bevorzugte nichtflüchtige Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethyl-hexylsuccinat und Di-(2-hexyldecyl)-succinat.

Weitere erfindungsgemäß besonders bevorzugte nichtflüchtige Öle sind ausgewählt aus den symmetrischen, unsymmetrischen oder cyclischen Estern der Kohlensäure mit C₆-C₂₀-Alkoholen, z. B. Dicaprylylcarbonat (z. B. Cetiol^{®} CC) oder Di-(2-ethylhexyl)carbonat.

Weitere erfindungsgemäß besonders bevorzugte nichtflüchtige Öle sind ausgewählt aus nichtflüchtigen Siliconölen. Hierzu zählen insbesondere lineare Polydimethylsiloxane mit einer kinematischen Viskosität bei 25°C von mindestens bis 2000 cSt, wie sie z. B. unter Dow Corning^{®} 200 bzw. Xiameter PMX von Dow Corning erhältlich sind. Weitere bevorzugte nichtflüchtige Siliconöle sind Phenyltrimethicone mit einer kinematischen Viskosität bei 25°C von mindestens 10 bis 100 cSt, bevorzugt von 15 - 30 cSt sowie Cetyldimethicone.

Weitere erfindungsgemäß besonders bevorzugte nichtflüchtige Öle sind ausgewählt aus nichtflüchtigen aliphatischen Alkanen und Alkenen, wie insbesondere 1,3-Bis(2-ethylhexyl)cyclohexan, Polydecene, hydrierte Polydecene, Polyisobutene, hydrierte Polyisobutene, Eicosan, Isoeicosan, Squalan und Squalen.

Weitere nichtflüchtige Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere erfindungsgemäß besonders bevorzugte nichtflüchtige Öle sind solche, die einen Brechungsindex n_{D}²⁰ von mindestens 1,44, bevorzugt von 1,445 bis 1,54 aufweisen. Derartige Öle sind besonders gut geeignet, sichtbare Rückstände des Antitranspirant-Wirkstoffs auf der Haut oder auf der Kleidung zu maskieren.

In der nachfolgenden Tabelle 1 sind erfindungsgemäß bevorzugte nichtflüchtige Öle mit einem Brechungsindex n_{D}²⁰ von mindestens 1,44 aufgeführt.

**Tabelle 1: Bevorzugte nichtflüchtige Öle mit einem Brechungsindex n_{D}²⁰ von mindestens 1,44**

| Nicht-flüchtiges Öl | n_{D}²⁰ |
|---|---|
| PPG-2 Myristyl ether propionate | 1,4408 |
| PPG-3 Myristyl ether | 1,4438 |
| Lauryllactat | 1,4448 |
| Hydrogenated Polydecene (75-85 Gew.-% C30 und 25-15 Gew.-% C40) | 1,445 |
| PPG-14 butylether | 1,4460 |
| Glyceryltri(2-ethylhexanoat), Tri(2-ethylhexanoin) | 1,4467 |
| 2-Ethylhexylpalmitat | 1,447 |
| PPG-15 butylether | 1,4479 |
| 2-Ethylhexylstearat | 1,448 |
| Dow Corning 2502 (Cetyl Dimethicone) | 1,4488 |
| Cetearylisononanoat | 1,449 |
| Caprylic/Capric Triglycerides | 1,4492 |
| 2-Hexyldecanol | 1,45 |
| PPG-53 butylether | 1,4512 |
| Isostearyllactat | 1,4519 |
| Cetiol PGL (Hexyldecanol, Hexyldecyl laurate) | 1,4525 |
| Isocetylpalmitat | 1,4538 |
| Isocetylstearat | 1,4541 |
| Isostearylpalmitat | 1,4546 |
| 2-Octyldodecanol | 1,455 |
| 2-Hexyldecylstearat | 1,456 |
| Isostearylalkohol | 1,4578 |
| 1,3-Bis(2-ethylhexyl)cyclohexan | 1,4598 |
| Dow Corning 556 Fluid (Phenyl Trimethicone) | 1,460 |
| Isostearylisostearat | 1,4612 |
| Oleylerucat | 1,467 |
| Benzyllaurat | 1,4811 |
| C12-C15 Alkylbenzoat | 1,4820 |
| Isostearylbenzoat | 1,4820 |
| 2-Octyldodecylbenzoat | 1,4833 |
| Glycereth-7 benzoat | 1,4953 |

Erfindungsgemäß bevorzugte Antitranspirant-Stifte enthalten, jeweils bezogen auf das Gesamtgewicht des Stifts, 1 - 30 Gew.-%, bevorzugt 5 - 25 Gew.-%, besonders bevorzugt 8 - 20 Gew.-%, außerordentlich bevorzugt 10 - 15 Gew.-%, mindestens eines nichtflüchtigen Öls mit einem Brechungsindex n_{D}²⁰ von mindestens 1,44, bevorzugt von 1,445 bis 1,54.

### Wachskomponente

Die erfindungsgemäßen Antitranspirant-Stifte enthalten insgesamt 15 - 25 Gew.-% mindestens einer unter Normalbedingungen festen Wachskomponente, wobei insgesamt 12 - 22 Gew.-% mindestens eines linearen und gesättigten C₁₄-C₂₂-Alkanols, 0,5 - 5 Gew.-% mindestens eines Wachses mit einem Schmelzpunkt von 65 - 150 °C und 0,5 - 8 Gew.-% mindestens eines Wachses mit einem Schmelzpunkt von 25 bis < 50°C enthalten sind.

Im Sinne der vorliegenden Anmeldung sind unter Wachskomponenten, die unter Normalbedingungen fest sind, lineare und gesättigte C₁₄-C₂₂-Alkanole, natürliche pflanzliche und tierische Wachse sowie deren synthetische Nachbildungen, wie z. B. synthetisches Bienenwachs, zu verstehen, sowie weiterhin die Wachse, die nachstehend explizit als geeignete Wachskomponenten genannt sind.

Weitere Komponenten, die unter Normalbedingungen fest sind, zum Beispiel ethoxylierte Alkanole, wie Ceteareth-12, zählen nicht zu den Wachskomponenten. Auch unter Normalbedingungen feste Alkansäuren zählen nicht zu den Wachskomponenten.

Als unter Normalbedingungen feste Wachskomponente enthalten die erfindungsgemäßen Stifte, jeweils bezogen auf ihr Gesamtgewicht, insgesamt 12 - 22 Gew.-%, bevorzugt 13 - 20 Gew.-%, besonders bevorzugt 14 - 19 Gew.-%, außerordentlich bevorzugt 15 - 18 Gew.-%, mindestens eines linearen und gesättigten C₁₄-C₂₂-Alkanols. Bevorzugte C₁₄-C₂₂-Alkanole sind ausgewählt aus Myristylalkohol, Cetylalkohol, Stearylalkohol, Arachidylalkohol und Behenylalkohol sowie Mischungen hiervon. Besonders bevorzugt sind Cetylalkohol, Stearylalkohol sowie Mischungen hiervon. Außerordentlich bevorzugt ist Stearylalkohol. Weiter bevorzugt ist die alleinige Verwendung von Stearylalkohol. Weitere erfindungsgemäß bevorzugte Stifte enthalten, jeweils bezogen auf ihr Gesamtgewicht, insgesamt 12 - 22 Gew.-%, einer Mischung aus zwei oder drei linearen und gesättigten C₁₄-C₂₂-Alkanolen, wobei 0,1 - 3 Gew.-%, bezogen auf den gesamten Stift, Arachidylalkohol und/oder Behenylalkohol enthalten sind und das restliche C₁₄-C₂₂-Alkanol aus Stearylalkohol besteht.

Zusätzlich zu den vorstehend beschriebenen C₁₄-C₂₂-Alkanolen enthalten die erfindungsgemäßen Stifte, jeweils bezogen auf ihr Gesamtgewicht, insgesamt 0,5 - 5 Gew.-%, bevorzugt 1 - 4 Gew.-%, besonders bevorzugt 1,5 - 2 Gew.-%, mindestens eines Wachses mit einem Schmelzpunkt von 65 - 150 °C.

Erfindungsgemäß bevorzugte Wachse mit einem Schmelzpunkt von 65 - 150 °C sind natürliche pflanzliche Wachse, insbesondere Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse und Grapefruitwachs, und tierische Wachse, insbesondere Bienenwachs, Schelllackwachs und Walrat, sowie synthetische Nachbildungen natürlicher Wachse, wie z. B. synthetisches Bienenwachs. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, bevorzugt. Zu den synthetischen Wachsen, die ebenfalls erfindungsgemäß bevorzugt sind, zählen Polyalkylenwachse, insbesondere Polyethylenwachse, Paraffinwachse und Polyethylenglycole, die nur Polyethylenglycol-Einheiten im Molekül aufweisen und ein Molgewicht von mindestens 3 Millionen Dalton aufweisen, insbesondere Verbindungen mit der INCI-Bezeichnung PEG-90M, C₂₀-C₄₀-Dialkylester von Dimersäuren, C₃₀₋₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie der Antitranspirantzubereitung ausgezeichnete sensorische Eigenschaften und dem Stift insgesamt eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt von 65 - 150°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt von 65 - 150°C haben. Insbesondere können als Wachskomponente C₁₆₋₃₆-Alkylstearate und C₁₈₋₃₈-Alkylhydroxystearoylstearate, C₂₀₋₄₀-Alkylerucate, Cetylbehenat und Stearylbehenat vorteilhaft sein. Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben.

Weitere besonders bevorzugte Wachskomponenten mit einem Schmelzpunkt von 65 - 150°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 65 - 150°C aufweisen, beispielsweise bevorzugt C₁₈ - C₃₆ Acid Triglyceride. Erfindungsgemäß ist als Wachskomponente hydriertes Rizinusöl, erhältlich z. B. als Handelsprodukt Cutina^{®} HR, welches besonders bevorzugt in einer Menge von 1 - 4 Gew.-%, besonders bevorzugt 1,5 - 2 Gew.-%, jeweils bezogen auf das Gesamtgewicht des Stiftes, enthalten ist.

Erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% mindestens eines linearen und gesättigten C₁₄-C₂₂-Alkanols, ausgewählt aus Cetylalkohol, Stearylalkohol, Arachidylalkohol und Behenylalkohol sowie Mischungen hiervon, insgesamt 0,5 - 5 Gew.-% mindestens eines Wachses mit einem Schmelzpunkt von 65 - 150 °C und 0,5 - 8 Gew.-% mindestens eines Wachses mit einem Schmelzpunkt von 25 bis < 50°C enthalten sind.

Weitere erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% eines linearen und gesättigten C₁₄-C₂₂-Alkanols, das nur aus Stearylalkohol ausgewählt ist, weiterhin insgesamt 0,5 - 5 Gew.-% mindestens eines Wachses mit einem Schmelzpunkt von 65 - 150 °C und 0,5 - 8 Gew.-% mindestens eines Wachses mit einem Schmelzpunkt von 25 bis < 50°C enthalten sind.

Weitere erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% einer Mischung aus zwei oder drei linearen und gesättigten C₁₄-C₂₂-Alkanolen, wobei 0,1 - 3 Gew.-%, bezogen auf den gesamten Stift, Arachidylalkohol und/oder Behenylalkohol enthalten sind und das restliche C₁₄-C₂₂-Alkanol aus Stearylalkohol besteht, weiterhin insgesamt 0,5 - 5 Gew.-% mindestens eines Wachses mit einem Schmelzpunkt von 65 - 150 °C und 0,5 - 8 Gew.-% mindestens eines Wachses mit einem Schmelzpunkt von 25 bis < 50°C enthalten sind.

Erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% mindestens eines linearen und gesättigten C₁₄-C₂₂-Alkanols, ausgewählt aus Cetylalkohol, Stearylalkohol, Arachidylalkohol und Behenylalkohol sowie Mischungen hiervon, insgesamt 0,5 - 5 Gew.-% mindestens eines Wachses mit einem Schmelzpunkt von 65 - 150 °C, ausgewählt aus hydriertem Rizinusöl, und 0,5 - 8 Gew.-% mindestens eines Wachses mit einem Schmelzpunkt von 25 bis < 50°C enthalten sind.

Weitere erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% eines linearen und gesättigten C₁₄-C₂₂-Alkanols, das nur aus Stearylalkohol ausgewählt ist, weiterhin insgesamt 0,5 - 5 Gew.-% mindestens eines Wachses mit einem Schmelzpunkt von 65 - 150 °C, ausgewählt aus hydriertem Rizinusöl, und 0,5 - 8 Gew.-% mindestens eines Wachses mit einem Schmelzpunkt von 25 bis < 50°C enthalten sind.

Weitere erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% einer Mischung aus zwei oder drei linearen und gesättigten C₁₄-C₂₂-Alkanolen, wobei 0,1 - 3 Gew.-%, bezogen auf den gesamten Stift, Arachidylalkohol und/oder Behenylalkohol enthalten sind und das restliche C₁₄-C₂₂-Alkanol aus Stearylalkohol besteht, weiterhin insgesamt 0,5 - 5 Gew.-% mindestens eines Wachses mit einem Schmelzpunkt von 65 - 150 °C, ausgewählt aus hydriertem Rizinusöl, und 0,5 - 8 Gew.-% mindestens eines Wachses mit einem Schmelzpunkt von 25 bis < 50°C enthalten sind.

Zusätzlich zu den vorstehend beschriebenen C₁₄-C₂₂-Alkanolen und den Wachsen mit einem Schmelzpunkt von 65 - 150 °C enthalten die erfindungsgemäßen Stifte, jeweils bezogen auf ihr Gesamtgewicht, insgesamt 0,5 - 8 Gew.-%, bevorzugt 1 - 6 Gew.-%, besonders bevorzugt 3 - 4 Gew.-%, mindestens eines Wachses mit einem Schmelzpunkt von 25 bis < 50°C.

Bevorzugte Wachse mit einem Schmelzpunkt im Bereich von 25 - < 50°C sind ausgewählt aus Kokosfettsäureglycerinmono-, -di- und -triestern, die teilgehärtet oder vollständig gehärtet sein können, Mischungen aus Kokosfettsäureglycerinmono-, -di- und -triestern, Mischungen aus teilgehärteten oder vollständig gehärteten Kokosfettsäureglycerinmono-, -di- und -triestern, teilgehärteten oder vollständig gehärteten pflanzlichen Ölen, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen. Diese im Bereich von 25 - < 50°C schmelzenden Wachskomponenten ermöglichen eine Konsistenzoptimierung des Produktes und eine Minimierung der sichtbaren Rückstände auf der Haut. Besonders bevorzugt sind Kokosfettsäureglyceride, insbesondere solche mit der INCI-Bezeichnung Cocoglycerides, insbesondere die Handelsprodukte Novata^{®} (ex BASF/Cognis), besonders bevorzugt ein Gemisch aus C₁₂-C₁₈-Mono-, Di- und Triglyceriden, das im Bereich von 30 - 32°C schmilzt, wie es beispielsweise unter dem Handelsnamen Novata^{®} AB von BASF/Cognis erhältlich ist, sowie gehärtete und teilgehärtete Kokosfettsäureglyceride, insbesondere solche mit der INCI-Bezeichnung Hydrogenated Cocoglycerides, insbesondere erhältlich als Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₈-C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol^{®} MM). Besonders bevorzugte Wachse mit einem Schmelzpunkt im Bereich von 25 - < 50°C sind ausgewählt aus Mischungen aus Kokosfettsäureglycerinmono-, -di- und -triestern, Mischungen aus teilgehärteten oder vollständig gehärteten Kokosfettsäureglycerinmono-, -di- und -triestern, teilgehärteten oder vollständig gehärteten pflanzlichen Ölen, Butyrospermum Parkii (Shea Butter), Stearyllaurat, Cetearylstearat, Cetylpalmitat und Myristylmyristat sowie Mischungen hiervon.

Erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% mindestens eines linearen und gesättigten C₁₄-C₂₂-Alkanols, ausgewählt aus Cetylalkohol, Stearylalkohol, Arachidylalkohol und Behenylalkohol sowie Mischungen hiervon, insgesamt 0,5 - 5 Gew.-% mindestens eines Wachses mit einem Schmelzpunkt von 65 - 150 °C, ausgewählt aus hydriertem Rizinusöl, und 0,5 - 8 Gew.-% mindestens eines Wachses mit einem Schmelzpunkt von 25 bis < 50°C, ausgewählt aus Mischungen aus Kokosfettsäureglycerinmono-, -di- und -triestern, Mischungen aus teilgehärteten oder vollständig gehärteten Kokosfettsäureglycerinmono-, -di- und -triestern, teilgehärteten oder vollständig gehärteten pflanzlichen Ölen, Butyrospermum Parkii (Shea Butter), Stearyllaurat, Cetearylstearat, Cetylpalmitat und Myristylmyristat sowie Mischungen hiervon, enthalten sind.

Weitere erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% eines linearen und gesättigten C₁₄-C₂₂-Alkanols, das nur aus Stearylalkohol ausgewählt ist, weiterhin insgesamt 0,5 - 5 Gew.-% mindestens eines Wachses mit einem Schmelzpunkt von 65 - 150 °C, ausgewählt aus hydriertem Rizinusöl, und 0,5 - 8 Gew.-% mindestens eines Wachses mit einem Schmelzpunkt von 25 bis < 50°C, ausgewählt aus Mischungen aus Kokosfettsäureglycerinmono-, -di- und -triestern, Mischungen aus teilgehärteten oder vollständig gehärteten Kokosfettsäureglycerinmono-, -di- und -triestern, teilgehärteten oder vollständig gehärteten pflanzlichen Ölen, Butyrospermum Parkii (Shea Butter), Stearyllaurat, Cetearylstearat, Cetylpalmitat und Myristylmyristat sowie Mischungen hiervon, enthalten sind.

Weitere erfindungsgemäß bevorzugte Stifte enthalten, jeweils bezogen auf ihr Gesamtgewicht, insgesamt 15 - 25 Gew.-% mindestens einer unter Normalbedingungen festen Wachskomponente, wobei insgesamt 12 - 22 Gew.-% einer Mischung aus zwei oder drei linearen und gesättigten C₁₄-C₂₂-Alkanolen, wobei 0,1 - 3 Gew.-%, bezogen auf den gesamten Stift, Arachidylalkohol und/oder Behenylalkohol enthalten sind und das restliche C₁₄-C₂₂-Alkanol aus Stearylalkohol besteht, weiterhin insgesamt 0,5 - 5 Gew.-% mindestens eines Wachses mit einem Schmelzpunkt von 65 - 150 °C, ausgewählt aus hydriertem Rizinusöl, und 0,5 - 8 Gew.-% mindestens eines Wachses mit einem Schmelzpunkt von 25 bis < 50°C, ausgewählt aus Mischungen aus Kokosfettsäureglycerinmono-, -di- und -triestern, Mischungen aus teilgehärteten oder vollständig gehärteten Kokosfettsäureglycerinmono-, -di- und -triestern, teilgehärteten oder vollständig gehärteten pflanzlichen Ölen, Butyrospermum Parkii (Shea Butter), Stearyllaurat, Cetearylstearat, Cetylpalmitat und Myristylmyristat sowie Mischungen hiervon, enthalten sind.

### Füllstoff

Die erfindungsgemäßen Stifte enthalten zur Verbesserung der Stiftkonsistenz und der sensorischen Eigenschaften, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% mindestens eines wasserunlöslichen teilchenförmigen Füllstoffs. Erfindungsgemäß bevorzugte wasserunlösliche teilchenförmige Füllstoffe sind ausgewählt aus Silicaten, vor allem Schichtsilicaten und unter diesen insbesondere Montmorillonit, Kaolinit, Ilit, Beidellit, Nontronit, Saponit, Hectorit, Bentonit, Smectit und Talkum, weiterhin Siliciumdioxid, Kieselsäuren, z. B. Aerosil^{®}-Typen, Polyalkylsesquisiloxan-Partikeln (insbesondere Aerosil^{®} R972 und Aerosil^{®} 200V von Degussa), Kieselgelen, Silica, Cellulosepulvern, Stärken, modifizierten Stärken (z. B. aus Mais, Reis, Kartoffeln) und Stärkederivaten, die vorverkleistert sein können, Kaolin, Magnesiumaluminiumsilikaten, Zeolithen, Perlit (vulkanischem Glas), Blähperlit, Bornitrid, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Besonders bevorzugte wasserunlösliche teilchenförmige Füllstoffe sind ausgewählt aus Talkum, Siliciumdioxid, Kieselsäuren, z. B. Aerosil^{®}-Typen, Polyalkylsesquisiloxan-Partikeln (insbesondere Aerosil^{®} R972 und Aerosil^{®} 200V von Degussa), Kieselgelen, Silica, Perlit, Blähperlit, sowie Mischungen dieser Substanzen.

Außerordentlich bevorzugte erfindungsgemäße Stifte enthalten, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% mindestens eines wasserunlöslichen teilchenförmigen Füllstoffs, ausgewählt aus Talkum, Siliciumdioxid, Kieselsäuren, Polyalkylsesquisiloxan-Partikel, Kieselgel, Silica, sowie Mischungen dieser Substanzen. Weitere außerordentlich bevorzugte erfindungsgemäße Stifte enthalten, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% mindestens eines wasserunlöslichen teilchenförmigen Füllstoffs, ausgewählt aus Talkum. Weitere außerordentlich bevorzugte erfindungsgemäße Stifte enthalten, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% Talkum als einzigem wasserunlöslichen teilchenförmigen Füllstoff.

### Fettalkoholethoxylate

Die erfindungsgemäßen Stifte enthalten, jeweils bezogen auf ihr Gesamtgewicht, insgesamt 1 - 4 Gew.-%, bevorzugt 2 - 3,5 Gew.-%, besonders bevorzugt 2,5 - 3 Gew.-%, mindestens eines Öl-in-Wasser-Emulgators, der ausgewählt ist aus Ethoxylaten von C₁₀-C₂₂-Alkanolen mit 8 bis 16 Ethylenoxid-Einheiten im Molekül, bevorzugt mit 10 - 14 Ethylenoxid-Einheiten, bevorzugt mit 12 Ethylenoxid-Einheiten im Molekül. Überraschend wurde festgestellt, dass ein solcher Öl-in-Wasser-Emulgator in den beanspruchten Mengenbereichen verhindert, dass Feuchtigkeit, insbesondere Luftfeuchtigkeit, Hautfeuchtigkeit und Schweiß, zu einer Verhärtung, Versprödung oder sonstigen strukturellen Veränderung der Stiftoberfläche führt. Der erfindungsgemäße Öl-in-Wasser-Emulgator in den beanspruchten Mengenbereichen bewirkt, dass die Abgabeeigenschaften, insbesondere das Anschmelzverhalten und Gleitvermögen des Stiftes beim Auftragen auf die Haut, aber auch die Freisetzung des Antitranspirantwirkstoffs und damit die Antitranspirantleistung, langzeitstabil erhalten bleiben. Bevorzugte Öl-in-Wasser-Emulgatoren, die Ethoxylate von C₁₀-C₂₂-Alkanolen mit 8 bis 16 Ethylenoxid-Einheiten im Molekül sind, sind ausgewählt aus Deceth-8, Deceth-10, Deceth-12, Deceth-14, Deceth-16, Undeceth-8, Undeceth-10, Undeceth-12, Undeceth-14, Undeceth-16, Laureth-8, Laureth-9, Laureth-10, Laureth-11, Laureth-12, Laureth-14, Laureth-16, Trideceth-8, Trideceth-9, Trideceth-10, Trideceth-12, Trideceth-14, Trideceth-16, Myreth-8, Myreth-10, Myreth-12, Myreth-14, Myreth-16, Ceteth-8, Ceteth-10, Ceteth-12, Ceteth-14, Ceteth-15, Ceteth-16, Steareth-10, Steareth-12, Steareth-14, Steareth-16, Ceteareth-8, Ceteareth-10, Ceteareth-12, Ceteareth-14, Ceteareth-16, Beheneth-8, Beheneth-10, Beheneth-12, Beheneth-14, Beheneth-16, sowie Mischungen hiervon. Besonders bevorzugt sind Ceteth-10, Ceteth-12, Ceteth-14, Steareth-10, Steareth-12, Steareth-14, Myreth-10, Myreth-12, Myreth-14, Trideceth-9, Trideceth-10, Trideceth-12, Laureth-10, Laureth-11, Laureth-12, sowie Mischungen hiervon. Außerordentlich bevorzugt sind Ceteth-10, Ceteth-12, Ceteth-14, Steareth-10, Steareth-12, Steareth-14, sowie Mischungen hiervon. Besonders bevorzugt ist die Mischung aus Ceteth-12 und Steareth-12, die auch als Ceteareth-12 bezeichnet wird.

Bevorzugte erfindungsgemäße Stifte enthalten, jeweils bezogen auf ihr Gesamtgewicht, insgesamt 1 - 4 Gew.-%, bevorzugt 2 - 3,5 Gew.-%, besonders bevorzugt 2,5 - 3 Gew.-%, an mindestens einem Öl-in-Wasser-Emulgator, der ausgewählt ist aus Ceteth-10, Ceteth-12, Ceteth-14, Steareth-10, Steareth-12, Steareth-14, sowie Mischungen hiervon.

Weitere bevorzugte erfindungsgemäße Stifte enthalten, jeweils bezogen auf ihr Gesamtgewicht, insgesamt 1 - 4 Gew.-%, bevorzugt 2 - 3,5 Gew.-%, besonders bevorzugt 2,5 - 3 Gew.-%, an mindestens einem Öl-in-Wasser-Emulgator, der ausgewählt ist aus Mischungen aus Ceteth-12 und Steareth-12. Weitere bevorzugte erfindungsgemäße Stifte enthalten, jeweils bezogen auf ihr Gesamtgewicht, insgesamt 1 - 4 Gew.-%, bevorzugt 2 - 3,5 Gew.-%, besonders bevorzugt 2,5 - 3 Gew.-%, an mindestens einem Öl-in-Wasser-Emulgator, der ausgewählt ist aus Mischungen aus Ceteth-12 und Steareth-12, wobei keine weiteren Öl-in-Wasser-Emulgatoren enthalten sind. In der nachfolgenden Tabelle 1a sind erfindungsgemäß bevorzugte und besonders bevorzugte Antitranspirant-Stiftzusammensetzungen Nr.1 bis Nr. 52 zusammengestellt. Alle Mengenangaben sind in Gew.-%, bezogen auf das Gesamtgewicht der Stiftzusammensetzung.

| | Nr. 1 | Nr. 2 | Nr. 3 | Nr. 4 |
|---|---|---|---|---|
| Antitranspirant-Wirkstoff (USP) | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) mind. ein flüchtiges Öl, ausgewählt aus flüchtigen cyclischen Siliconen, flüchtigen linearen Siliconen und flüchtigen aliphatischen Kohlenwasserstoffen sowie Mischungen hiervon | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) mind. ein lineares u. gesättigtes C₁₄-C₂₂-Alkanol | 12 - 22 | 13 - 20 | 14 - 19 | 15 - 18 |
| d)ii) mind. ein Wachs mit einem Smp. von 65-150°C | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| mind. ein Öl-in-Wasser-Emulgator, ausgewählt aus Ethoxylaten von C₁₀-C₂₂-Alkanolen mit 8 bis 16 Ethylenoxid-Einheiten im Molekül | 1 - 4 | 2 - 3,5 | 2,5 - 3 | 2,5 - 3 |

| | Nr. 5 | Nr. 6 | Nr. 7 | Nr. 8 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) mind. ein flüchtiges Öl, ausgewählt aus flüchtigen cyclischen Siliconen, flüchtigen linearen Siliconen und flüchtigen aliphatischen Kohlenwasserstoffen sowie Mischungen hiervon | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) mind. ein lineares u. gesättigtes C₁₄-C₂₂-Alkanol | 12 - 22 | 13 - 20 | 14 - 19 | 15 - 18 |
| d)ii) mind. ein Wachs mit einem Smp. von 65-150°C | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| mind. ein Öl-in-Wasser-Emulgator, ausgewählt aus Ethoxylaten von C₁₀-C₂₂-Alkanolen mit 8 bis 16 Ethylenoxid-Einheiten im Molekül | 1 - 4 | 2 - 3,5 | 2,5 - 3 | 2,5 - 3 |

| | Nr. 9 | Nr. 10 | Nr. 11 | Nr. 12 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) mind. ein flüchtiges Öl, ausgewählt aus flüchtigen cyclischen Siliconen, flüchtigen linearen Siliconen und flüchtigen aliphatischen Kohlenwasserstoffen sowie Mischungen hiervon | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) mind. ein lineares u. gesättigtes C₁₄-C₂₂-Alkanol | 12 - 22 | 13 - 20 | 14 - 19 | 15 - 18 |
| d)ii) mind. ein Wachs mit einem Smp. von 65-150°C | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| mind. ein Öl-in-Wasser-Emulgator, ausgewählt aus Ceteth-10, Ceteth-12, Ceteth-14, Steareth-10, Steareth-12, Steareth-14, sowie Mischungen hiervon | 1 - 4 | 2 - 3,5 | 2,5 - 3 | 2,5 - 3 |

| | Nr. 13 | Nr. 14 | Nr. 15 | Nr. 16 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) mind. ein lineares u. gesättigtes C₁₄-C₂₂-Alkanol | 12 - 22 | 13 - 20 | 14 - 19 | 15 - 18 |
| d)ii) mind. ein Wachs mit einem Smp. von 65-150°C | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| mind. ein Öl-in-Wasser-Emulgator, ausgewählt aus Ceteth-10, Ceteth-12, Ceteth-14, Steareth-10, Steareth-12, Steareth-14, sowie Mischungen hiervon | 1 - 4 | 2 - 3,5 | 2,5 - 3 | 2,5 - 3 |

| | Nr. 17 | Nr. 18 | Nr. 19 | Nr. 20 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| mind. ein Öl-in-Wasser-Emulgator, ausgewählt aus Ceteth-10, Ceteth-12, Ceteth-14, Steareth-10, Steareth-12, Steareth-14, sowie Mischungen hiervon | 1 - 4 | 2 - 3,5 | 2,5 - 3 | 2,5 - 3 |

| | Nr. 21 | Nr. 22 | Nr. 23 | Nr.24 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| Öl-in-Wasser-Emulgator, ausgewählt aus Mischungen von Ceteth-12 und Steareth-12 | 1 - 4 | 2 - 3,5 | 2,5 - 3 | 2,5 - 3 |

| | Nr. 25 | Nr. 26 | Nr. 27 | Nr. 28 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 25 - 30 |
| c)ii) mind. ein nicht-flüchtiges Öl, ausgewählt aus PPG-14 Butylether | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| Talkum | 0,5 - 6 | 1 - 5 | 2 - 4 | 3 - 3,5 |
| Öl-in-Wasser-Emulgator, ausgewählt aus Mischungen von Ceteth-12 und Steareth-12 | 1 - 4 | 2 - 3,5 | 2,5 - 3 | 2,5 - 3 |

| | Nr. 29 | Nr. 30 | Nr. 31 | Nr. 32 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 25 - 30 |
| c)ii) mind. ein nicht-flüchtiges Öl, ausgewählt aus PPG-14 Butylether | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| Talkum | 0,5 - 6 | 1 - 5 | 2 - 4 | 3 - 3,5 |
| Öl-in-Wasser-Emulgator, ausgewählt aus Mischungen von Ceteth-12 und Steareth-12, wobei keine weiteren Öl-in-Wasser-Emulgatoren enthalten sind | 1 - 4 | 2 - 3,5 | 2,5 - 3 | 2,5 - 3 |

| | Nr. 33 | Nr. 34 | Nr. 35 | Nr. 36 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 20 - 35 |
| c)ii) mind. ein nicht-flüchtiges Öl, ausgewählt aus PPG-14 Butylether | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 -5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| Talkum | 0,5 - 6 | 1 - 5 | 2 - 4 | 3 - 3,5 |
| Öl-in-Wasser-Emulgator, ausgewählt aus Mischungen von Ceteth-12 und Steareth-12, wobei keine weiteren Öl-in-Wasser-Emulgatoren enthalten sind | 1 - 4 | 2 - 3,5 | 2,5 - 3 | 2,5 - 3 |

| | Nr. 37 | Nr. 38 | Nr. 39 | Nr. 40 |
|---|---|---|---|---|
| Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 20 - 35 |
| c)ii) mind. ein nicht-flüchtiges Öl, ausgewählt aus PPG-14 Butylether | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| Talkum | 0,5 - 6 | 1 - 5 | 2 - 4 | 3 - 3,5 |
| Öl-in-Wasser-Emulgator, ausgewählt aus Mischungen von Ceteth-12 und Steareth-12, wobei keine weiteren Öl-in-Wasser-Emulgatoren enthalten sind | 1 - 4 | 2 - 3,5 | 2,5 - 3 | 2,5 - 3 |

| | Nr. 41 | Nr. 42 | Nr. 43 | Nr. 44 |
|---|---|---|---|---|
| Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 20 - 35 |
| c)ii) mind. ein nicht-flüchtiges Öl, ausgewählt aus PPG-14 Butylether | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C, ausgewählt aus Mischungen aus Kokosfettsäureglycerinmono-, -di- und -triestern, Mischungen aus teilgehärteten oder vollständig gehärteten Kokosfettsäureglycerinmono-, -di-und -triestern, teilgehärteten oder vollständig gehärteten pflanzlichen Ölen, Butyrospermum Parkii, Stearyllaurat, Cetearylstearat, Cetylpalmitat und Myristylmyristat sowie Mischungen hiervon | 0,5 - 8 | 1 - 6 | 3 - 4 | 3 - 4 |
| Talkum | 0,5 - 6 | 1 - 5 | 2 - 4 | 3 - 3,5 |
| ein Öl-in-Wasser-Emulgator, ausgewählt aus Mischungen von Ceteth-12 und Steareth-12, wobei keine weiteren Öl-in-Wasser-Emulgatoren enthalten sind | 1 - 4 | 2 - 3,5 | 2,5 - 3 | 2,5- 3 |

| | Nr. 45 | Nr. 46 | Nr. 47 | Nr. 48 |
|---|---|---|---|---|
| Aluminiumchlorhydrat oder Aluminiumzirconiumchlorhydrat, insbesondere Aluminium-Zirconium-Tetrachlorohydrex-Glycin-Komplex | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 20 - 35 |
| c)ii) mind. ein nicht-flüchtiges Öl, ausgewählt aus Benzoesäure-C₁₂-C₁₅-Alkylester | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C, ausgewählt aus Mischungen aus Kokosfettsäureglycerinmono-, -di- und -triestern, Mischungen aus teilgehärteten oder vollständig gehärteten Kokosfettsäureglycerinmono-, -di-und -triestern, teilgehärteten oder vollständig gehärteten pflanzlichen Ölen, Butyrospermum Parkii, Stearyllaurat, Cetearylstearat, Cetylpalmitat und Myristylmyristat sowie Mischungen hiervon | 0,5 - 8 | 1 - 6 | 3 - 4 | 3 - 4 |
| Talkum | 0,5 - 6 | 1 - 5 | 2 - 4 | 3 - 3,5 |
| ein Öl-in-Wasser-Emulgator, ausgewählt aus Mischungen von Ceteth-12 und Steareth-12, wobei keine weiteren Öl-in-Wasser-Emulgatoren enthalten sind | 1 - 4 | 2 - 3,5 | 2,5 - 3 | 2,5 - 3 |

| | Nr. 49 | Nr. 50 | Nr. 51 | Nr. 52 |
|---|---|---|---|---|
| ein adstringierendes Aluminiumsalz mit zahlenmittl. Partikelgröße von 1 - 50 µm, bevorzugt 3 - 20 µm, besonders bevorzugt 5 - 10 µm, ausgewählt aus Aluminiumchlorhydrat, Aluminiumsesquichlorohydrat, Aluminium-Zirconium-Tetrachlorohydrex-Glycin-Komplexen, Aluminium-Zirconiumpentachlorohydex-Glycin-Komplexen u. Aluminium-Zirconiumoctachlorohydex-Glycin-Komplexen sowie Mischungen hiervon | 3 - 27 | 5 - 22 | 10 - 20 | 10 - 20 |
| freies Wasser | 0 - 8 | 0,1 - 6 | 0,2 - 4 | 0,5 - 3 |
| insgesamt mind. ein unter Normalbedingungen flüssiges kosmetisches Öl c) | 30 - 70 | 30 - 70 | 30 - 70 | 30 - 70 |
| c)i) Cyclomethicone | 10 - 50 | 15 - 40 | 20 - 35 | 25 - 30 |
| c)ii) mind. ein nicht-flüchtiges Öl | 1 - 30 | 5 - 25 | 8 - 20 | 10 - 15 |
| d) mind. eine unter Normalbedingungen feste Wachskomponente, darin | 15 - 25 | 15 - 25 | 15 - 25 | 15 - 25 |
| d)i) Stearylalkohol | 12 - 22 | 13 - 20 | 14 - 19 | 15 - 18 |
| d)ii) hydriertes Rizinusöl | 0,5 - 5 | 1 - 4 | 1,5 - 2 | 1,5 - 2 |
| d)iii) mind. ein Wachs mit Smp. von 25 bis < 50°C | 0,5 - 8 | 1 - 6 | 3 - 4 | 3 - 4 |
| mind. ein wasserunlöslicher teilchenförmiger Füllstoff | 0,5 - 6 | 1 - 5 | 2 - 4 | 3 - 3,5 |
| Öl-in-Wasser-Emulgator, ausgewählt aus Mischungen von Ceteth-12 und Steareth-12 | 1 - 4 | 2 - 3,5 | 2,5 - 3 | 2,5 - 3 |

Die erfindungsgemäßen Stifte enthalten bevorzugt mindestens einen Riechstoff, besonders bevorzugt in einer Gesamtmenge von 0,1 bis 5 Gew.-%, außerordentlich bevorzugt 0,3 - 2 Gew.-%, jeweils bezogen auf das Gesamtgewicht des Stiftes.

Um die schweißhemmende und geruchsreduzierende Wirkung der erfindungsgemäßen Antitranspirant-Stifte weiter zu verbessern, enthalten diese in einer bevorzugten Ausführungsform mindestens einen Deodorant-Wirkstoff.

Erfindungsgemäß bevorzugte Deodorant-Wirkstoffe sind keimhemmende Mittel, präbiotisch wirksame Komponenten sowie Enzyminhibitoren oder, besonders bevorzugt, Kombinationen der genannten Wirkstoffe.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in DE 10333245 und DE 102004011968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Weitere bevorzugte Deodorant-Wirkstoffe sind ausgewählt aus den keimhemmend wirkenden Parfümölen und den Deosafe^{®}-Parfümölen, die von der Firma Symrise, vormals Haarmann und Reimer, erhältlich sind.

Weitere bevorzugte Deodorant-Wirkstoffe sind ausgewählt aus Silbersalzen, insbesondere Silbercitrat, Dihydrogensilbercitrat, Silberlactat und Silbersulfat, löslichen Komplexsalzen des Silbers, kolloidalem Silber und Silberzeolithen.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugte erfindungsgemäße Antitranspirant-Stifte sind dadurch gekennzeichnet, dass der mindestens eine Deodorant-Wirkstoff ausgewählt ist aus Arylsulfatase-Inhibitoren, beta-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere alpha-(2-Ethylhexyl)glycerinether, Phenoxyethanol, keimhemmend wirkenden Parfümölen, Cyclodextrinen, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Silbersalzen, Silberzeolithen sowie Mischungen der vorgenannten Substanzen.

Weitere bevorzugte erfindungsgemäße Antitranspirant-Stifte enthalten, jeweils bezogen auf ihr Gesamtgewicht, mindestens einen Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%.

Eine weitere bevorzugte Ausführungsform der Erfindung ist dadurch gekennzeichnet, dass 0,01 - 5 Gew.-%, bevorzugt 0,05 - 2 Gew.-%, besonders bevorzugt 0,1 - 1 Gew.-%, jeweils bezogen auf die gesamte erfindungsgemäße Zusammensetzung, einer aus einem natürlichen Mineralwasser, einem Thermalwasser oder einem natürlichen Heilwasser erhaltenen Mineralstoffmischung enthalten ist. Überraschend wurde festgestellt, dass derartige Mineralstoffmischungen die schweißhemmende Leistung der erfindungsgemäßen Zusammensetzungen weiter verbessern können. Außerdem können sich derartige Mineralstoffmischungen günstig auf die Hautverträglichkeit der erfindungsgemäßen Zusammensetzungen auswirken. Die Bezeichnung "natürliches Mineralwasser" richtet sich nach der Definition der deutschen Mineral- und Tafelwasserverordnung (MinTafWV, § 2). Als Mineralstoffmischung gilt der feste Rückstand der genannten Wässer. Erfindungsgemäß besonders bevorzugte Mineralstoffmischungen stammen aus dem Thermalwasser von La Toja (Spanien), Bad Blumau, Bad Radkersburg, Aachen, Wiesbaden (alle Deutschland), Karlsbad (Tschechien), La Bourboule, Enghien-les-bains, Allevard-les-bains, Digne, Nyrac-les-bains, Lons le Saunier, Eaux Bonnes, Rochefort, les Fumades, Saint Christau, Uriage-les-bains, la Roche-Posay (alle Frankreich) oder den natürlichen Mineralwässern oder Heilwässern von Evian, Volvic, Vichy, Avene, Vittel (alle Frankreich), Gerolstein oder Fachingen (Deutschland).

Überraschend wurde festgestellt, dass es die erfindungsgemäßen Antitranspirant-Stifte erlauben, bis zu 4 Gew.-%, bezogen auf das Gesamtgewicht des Stifts, an freiem Wasser einzuarbeiten, ohne an Lagerstabilität einzubüßen. Dies eröffnet die Möglichkeit, eine entsprechende Menge an hydrophilen Wirkstoffen in Form wässriger Lösungen oder Suspensionen einzuarbeiten. In einer bevorzugten Ausführungsform ist mindestens ein Deodorantwirkstoff als wässrige Lösung oder als wässrige Suspension enthalten, wobei die Einsatzmenge der Lösung so gewählt ist, dass der Antitranspirant-Stift, bezogen auf sein Gesamtgewicht, maximal 4 Gew.-% freies Wasser enthält.

Besonders bevorzugte erfindungsgemäße Antitranspirant-Stifte enthalten, bezogen auf ihr Gesamtgewicht, bevorzugt 0,2 - 4 Gew.-%, bevorzugt 0,3 - 2 Gew.-%, einer wässrigen Lösung mindestens eines Silbersalzes. In einer weiteren bevorzugten Ausführungsform ist mindestens ein Vitamin oder ein Provitamin als wässrige Lösung oder als wässrige Suspension enthalten, wobei die Einsatzmenge der Lösung so gewählt ist, dass der Antitranspirant-Stift, bezogen auf sein Gesamtgewicht, maximal 8 Gew.-% freies Wasser enthält. Diese Lehre lässt sich analog auch auf andere hydrophile Wirkstoffe, beispielsweise auf Haarwuchs inhibierende Wirkstoffe, Aminosäuren, Proteine und andere Wirkstoffe, übertragen.

Weiterhin können Konservierungsmittel, Farbstoffe, Antioxidanzien, insbesondere Tocopherole und Tocopherylester, Komplexbildner und andere Zusatzstoffe enthalten sein.

Ein weiterer Gegenstand der vorliegenden Anmeldung ist ein Verfahren zur nicht-therapeutischen Schweißreduktion, dass dadurch gekennzeichnet ist, dass eine erfindungsgemäße oder eine erfindungsgemäß bevorzugte Stiftzusammensetzung auf die Haut, insbesondere die Achselhaut, aufgetragen wird. Bezüglich weiterer bevorzugter Ausführungsformen des erfindungsgemäßen Verfahrens gilt mutatis mutandis das zu den erfindungsgemäßen Stiftzusammensetzungen Gesagte.

Ein weiterer Gegenstand der vorliegenden Anmeldung ist die Verwendung einer erfindungsgemäßen oder einer erfindungsgemäß bevorzugten Stiftzusammensetzung zur nicht-therapeutischen Schweißreduktion. Bezüglich weiterer bevorzugter Ausführungsformen der erfindungsgemäßen Verwendung gilt mutatis mutandis das zu den erfindungsgemäßen Stiftzusammensetzungen Gesagte.

Die nachfolgenden Formulierungsbeispiele sollen den Gegenstand der Erfindung erläutern, ohne ihn hierauf zu beschränken.

Es wurden folgende Antitranspirant-Stifte hergestellt (alle Mengenangaben in Gew.-%).

### Beispiel Nr. 3 ist erfindungsgemäß.

**Tabelle 2: Testformulierungen**

| | Nr. 1 (Vergleich) ohne O/W-Emulgator | Nr. 2 (Vergleich) 3 % PEG-40 stearat | Nr. 3 | Nr. 4 (Vergleich) | Nr. 5 (Vergleich) | Nr. 6 (Vergleich) |
|---|---|---|---|---|---|---|
| Cyclomethicone (mind. 95 Gew.-% Cyclopentasiloxan) | 35,2 | 32,2 | 32,2 | 30,2 | 40,7 | 54,7 |
| Hydrogenated Castor Oil | 1,5 | 1,5 | 1,5 | 1,5 | 2,0 | 2,0 |
| Stearylalkohol | 18 | 18 | 18 | 18 | 12 | 12 |
| Novata AB** | 4 | 4 | 4 | 4 | - | - |
| PPG-14 Butylether | 15,3 | 15,3 | 15,3 | 15,3 | - | - |
| PEG-40 stearat | - | 3 | - | - | - | - |
| Al-Zr-tetrachlorohydrex Gly* (USP) | 17,6 | 17,6 | 17,6 | 17,6 | - | - |
| Kristallwasser und (sofern vorhanden) Glycin | 4,4 | 4,4 | 4,4 | 4,4 | 6 | 3 |
| Talkum | 3 | 3 | 3 | 3 | 3 | 3 |
| Parfum | 1 | 1 | 1 | 1 | 0,3 | 1,3 |
| Eumulgin B1*** | - | - | 3 | 5 | 5 | 5 |
| Cetylalkohol | - | - | - | - | 5 | 5 |
| Aerosil R 972**** | - | - | - | - | 2 | 2 |
| Aluminiumchlorhydrat (USP) | - | - | - | - | 24 | 12 |

| | | | | | | |
|---|---|---|---|---|---|---|
| * zahlenmittlere Partikelgröße 3 - 20 µm ** INCI: Cocoglycerides, Schmelzpunkt 30 - 32 °C *** INCI: Ceteareth-12 *** INCI: Silica Dimethyl Silylate | | | | | | |

Die Stifte wurden unterschiedlichen Lagerungsbedingungen unterworfen und anschließend untersucht. Die Untersuchungsergebnisse sind in den folgenden Tabellen dargestellt.

**Tabelle 3: Stifteigenschaften nach einer Woche Lagerung bei 20 °C**

| Stift Nr. | Öl außen | Öl auf der Masse | Ölbindevermögen | † | Stiftmasse nach Abkühlen |
|---|---|---|---|---|---|
| 1 (V) | Nein | Ja-leicht | mittel | 1,5 | leicht gelblich; feine, glatte Oberfläche |
| 2 (V) | Nein | Ja-leicht | in Ordnung | 2,0 | leicht gelblich; feine, glatte Oberfläche |
| 3 (E) | Ja-mittel | Ja-mittel | leicht | 2,5 | in Ordnung, ölt auf Druck leicht aus |
| 4 (V) | Ja-stark | Nein | leicht | 2,3 | in Ordnung, ölt auf Druck leicht aus, sandig, stumpf |
| 5 (V) | Nein | Nein | in Ordnung | 1,5 | in Ordnung, ölt auf Druck leicht aus, sandig, stumpf |
| 6 (V) | nein | nein | mittel | 2,5 | in Ordnung, ölt auf Druck leicht aus, cremig |

| | | | | | |
|---|---|---|---|---|---|
| † Gesamtnote für Festigkeit, Anschmelzverhalten und Abgabeeigenschaften | | | | | |

Die Gesamtnote für Festigkeit, Anschmelzverhalten und Abgabeeigenschaften wird durch eine Gruppe geschulter Testpersonen nach den in Tabelle 4 dargestellten Kriterien bestimmt. Optimale Stifte erhalten die Note 2. Eine Veränderung dieser Beurteilung in Richtung 1 oder 0 bedeutet eine zunehmende Härte der Stiftoberfläche, eine Veränderung dieser Beurteilung in Richtung 3 bedeutet, dass die Stiftoberfläche weicher wird.

**Tabelle 4: Bewertung von Anschmelzverhalten und Abqabeeiqenschaften**

| **Gesamtnote** | **Anschmelzen** | **Abgabe** |
|---|---|---|
| 0 | kein Anschmelzen, keine Filmbildung | keine Abgabe, stumpfes Gleiten |
| 1 | träges Anschmelzen, zu hart, geringer Film | geringe Abgabe, geringes Gleiten |
| 2 | gutes Anschmelzen, Bildung eines seidigen Films | gute Abgabe, leichtes Gleiten |
| 3 | sehr leichtes Anschmelzen, zu weich | zu hohe Abgabe, zu cremig, schmiert |

**Tabelle 5: Stifteigenschaften nach Lagerung 1 Tag bei 40 °C, danach 2 Tage bei 45 °C**

| Stift Nr. | Öl außen | Öl auf der Masse | Ölbindevermögen | † | Stiftmasse nach Abkühlen |
|---|---|---|---|---|---|
| 1 (V) | ja-stark | ja-mittel | sehr schlecht | 1,5 | leicht gelblich; mittel-feine, kristalline Oberfläche |
| 2 (V) | ja-mittel | ja-mittel | schlecht | 1,5 | leicht gelblich; leicht-feine, kristalline Oberfläche |
| 3 (E) | nein | nein | mittel | 2,3 | ölt auf Druck aus |
| 4 (V) | ja-stark | ja-mittel | in Ordnung | 2,5 | ölt auf Druck leicht aus, sandig |
| 5 (V) | nein | nein | in Ordnung | 1,3 | hart, stumpf, schmiert |
| 6 (V) | ja-mittel | ja-leicht | in Ordnung | 2,8 | zu weich, schmiert |

In der nachstehenden Tabelle 6 sind die Testergebnisse der Untersuchungen der Teststifte auf ihre Langzeitstabilität gegenüber Feuchtigkeit hin dargestellt. Verglichen wurde die Sensorik unbenutzter Stifte, die nach der Herstellung 1 Woche bei 20 °C gelagert worden waren (= Reifezeit), mit der Sensorik von ebenso gereiften Stiften, für die 20 Anwendungszyklen simuliert worden waren (Simulation des Transfers von Feuchtigkeit und Schweiß auf den Stift).

**Tabelle 6: Langzeitstabilität gegenüber Feuchtigkeit**

| | unbenutzter Stift nach Reifezeit | | gereifter Stift nach 20 simulierten Anwendungszyklen | |
|---|---|---|---|---|
| Stift Nr. | † | Bemerkungen | † | Bemerkungen |
| 1 (V) | 2,3 | in Ordnung; ölt auf Druck aus | 1,3 | harte Oberfläche mit Stippenbildung, ölt auf Druck aus |
| 2 (V) | 1,8 | in Ordnung; ölt auf Druck aus, sandig | 1,0 | harte Oberfläche, ölt auf Druck aus, sandig, stumpf |
| 3 (E) | 2,0 | in Ordnung; ölt auf Druck leicht aus, cremig | 1,8 | ölt auf Druck aus |
| 4 (V) | 2,0 | in Ordnung; ölt auf Druck leicht aus, cremig | 1,3 | harte Oberfläche mit Stippenbildung, ölt auf Druck aus |
| 5 (V) | 1,5 | ölt auf Druck leicht aus, stumpf | 0,5 | sehr harter und stumpfer Stift |
| 6 (V) | 2,3 | ölt auf Druck leicht aus, cremig, sehrweich | 1,3 | ölt auf Druck aus, stumpfe Oberfläche |

Alle Vergleichsbeispiele zeigten unter Feuchtigkeitseinfluss eine starke Beeinträchtigung und Verschlechterung der Stiftoberfläche. Nur die Stiftoberfläche des erfindungsgemäßen Stiftes zeigte eine zufrieden stellende Langzeitstabilität.

In der nachstehenden Tabelle 7 sind die Testergebnisse der Untersuchungen der Teststifte auf die Veränderung der Härte der Stiftoberfläche unter Feuchtigkeitseinfluss hin dargestellt. Verglichen wurde die Härte der Stiftoberfläche unbenutzter Stifte, die nach der Herstellung 1 Woche bei 20 °C gelagert worden waren (= Reifezeit), mit der Härte der Stiftoberfläche von ebenso gereiften Stiften, für die 20 Anwendungszyklen simuliert worden waren (Simulation des Transfers von Feuchtigkeit und Schweiß auf den Stift). Die Härtemessung erfolgte durch Punktion der Oberfläche mit einem standardisierten Stahlkegel mit 45°. Registriert wurde das Gewicht, mit dem der Kegel belastet wurde, um bis zur Standardpenetrationstiefe von 5 mm in die Stiftoberfläche einzudringen. Es wurde der TEXTURE ANALYZER-1545 der Firma Stable Micro Systems verwendet. Die Vorschubgeschwindigkeit betrug 2,0 mm/s, Trigger value 1,0 g, PPS=200,0, Eindringtiefe 5,0 mm, Produkttemperatur 23,0 °C, relative Luftfeuchtigkeit 30%.

**Tabelle 7: Veränderung der Härte der Stiftoberfläche unter Feuchtigkeitseinfluss**

| | unbenutzter Stift nach Reifezeit | gereifter Stift nach 20 simulierten Anwendungszyklen |
|---|---|---|
| Stift Nr. | Gewicht für standardisierte Kegelpunktion der Stiftoberfläche [g] (Mittelwert aus 5 Messungen) | Gewicht für standardisierte Kegelpunktion der Stiftoberfläche [g] (Mittelwert aus 5 Messungen) |
| 1 (V) | 456 ± 10 | 440 ± 14 |
| 2 (V) | 645 ± 36 | 664 ± 35 |
| 3 (E) | 463 ± 27 | 448 ± 39 |
| 4 (V) | 572 ± 27 | 458 ± 8 |
| 5 (V) | 1107 ± 52 | 967 ± 15 |
| 6 (V) | 471 ± 26 | 432 ± 22 |

Die Vergleichsbeispiele 2 und 5 wiesen schon direkt nach Herstellung eine zu große Härte auf. Die für Vergleichsbeispiel 4 gemessene Erweichung der Stiftoberfläche war tatsächlich auf eine Versprödung der Stiftoberfläche zurückzuführen. Die Stiftoberfläche war krümelig, was bei der Penetrationsmessung einen niedrigen Gewichtswert hervorrief. Die Messung an Vergleichsbeispiel 6 lag im Bereich des erfindungsgemäßen Beispiels 3, allerdings wies Vergleichsbeispiel 6 keine befriedigende sensorische Gesamtnote auf, siehe Tabelle 6.

## Patentansprüche

1. Antitranspirant-Wachsstift, enthaltend
a) insgesamt 3 - 27 Gew.-%, bezogen auf das Gesamtgewicht der kristallwasserfreien Aktivsubstanz (USP) in der Gesamtzusammensetzung, Antitranspirant-Wirkstoff(e),
b) 0 - 4 Gew.-% freies Wasser,
c) insgesamt 30 - 70 Gew.-% an mindestens einem unter Normalbedingungen flüssigen kosmetischen Öl, darin
ci) 10 - 50 Gew.-% mindestens eines flüchtigen Öls, das ausgewählt ist aus flüchtigen cyclischen Siliconen, flüchtigen linearen Siliconen und flüchtigen aliphatischen Kohlenwasserstoffen sowie Mischungen hiervon, und
cii) zusätzlich zu dem mindestens einen flüchtigen Öl insgesamt 1 - 30 Gew.-% mindestens eines nicht-flüchtigen Öls,
d) insgesamt 15 - 25 Gew.-% mindestens einer unter Normalbedingungen festen Wachskomponente,
d)i. darin 12 - 22 Gew.-% mindestens eines linearen und gesättigten C₁₄-C₂₂-Alkanols,
d)ii. 0,5 - 5 Gew.-% mindestens eines Wachses mit einem Schmelzpunkt im Bereich von 65 - 150 °C,
d)iii. 0,5 - 8 Gew.-% mindestens eines Wachses mit einem Schmelzpunkt im Bereich von 25 bis < 50°C,
e) 0,5 - 6 Gew.-% mindestens eines wasserunlöslichen teilchenförmigen Füllstoffs,
f) 1 - 4 Gew.-%, bevorzugt 2 - 3,5 Gew.-%, besonders bevorzugt 2,5 - 3 Gew.-%, mindestens eines Öl-in-Wasser-Emulgators, ausgewählt aus Ethoxylaten von C₁₀-C₂₂-Alkanolen mit 8 bis 16 Ethylenoxid-Einheiten im Molekül,
wobei sich, sofern nichts anderes vermerkt ist, alle Gew.-%-Angaben auf das Gesamtgewicht des Antitranspirant-Wachsstifts beziehen.

2. Antitranspirant-Wachsstift gemäß Anspruch 1, **dadurch gekennzeichnet, dass** das mindestens eine flüchtige Öl in einer Gesamtmenge von 15 - 40 Gew.-%, besonders bevorzugt 20 - 35 Gew.-%, außerordentlich bevorzugt 25 - 30 Gew.-%, jeweils bezogen auf das Gesamtgewicht des Stifts, enthalten ist.

3. Antitranspirant-Wachsstift gemäß Anspruch 1 oder 2, **dadurch gekennzeichnet, dass** das mindestens eine flüchtige Öl ausgewählt ist aus Cyclopentasiloxan.

4. Antitranspirant-Wachsstift gemäß Anspruch 1, 2 oder 3, **dadurch gekennzeichnet, dass** das mindestens eine nicht-flüchtige Öl in einer Gesamtmenge von 5 - 25 Gew.-%, bevorzugt 8 - 20 Gew.-%, besonders bevorzugt 10 - 15 Gew.-%, enthalten ist, wobei sich alle Gew.-%-Angaben auf das Gesamtgewicht des Antitranspirant-Wachsstifts beziehen.

5. Antitranspirant-Wachsstift gemäß Anspruch 1, 2, 3 oder 4, **dadurch gekennzeichnet, dass** das mindestens eine nicht-flüchtige Öl einen Brechungsindex n_{D}²⁰ von mindestens 1,44, bevorzugt von 1,445 bis 1,54 aufweist.

6. Antitranspirant-Wachsstift gemäß Anspruch 1, 2, 3, 4 oder 5, **dadurch gekennzeichnet, dass** das mindestens eine nicht-flüchtige Öl ausgewählt ist aus mindestens einem Anlagerungsprodukts von 6 bis 20, bevorzugt 9 bis 15, Propylenoxid-Einheiten an ein ein- oder mehrwertiges C₃₋₂₂-Alkanol, besonders bevorzugt ausgewählt ist aus PPG-14 Butylether.

7. Antitranspirant-Wachsstift gemäß Anspruch 1, 2, 3, 4, 5 oder 6, **dadurch gekennzeichnet, dass** das mindestens eine nicht-flüchtige Öl, jeweils bezogen auf das Gesamtgewicht des Stifts, in einer Gesamtmenge von 5 - 25 Gew.-%, bevorzugt 8 - 20 Gew.-%, besonders bevorzugt 10 - 15 Gew.-%, enthalten ist.

8. Antitranspirant-Wachsstift gemäß Anspruch 1, 2, 3, 4, 5, 6 oder 7, **dadurch gekennzeichnet, dass** das mindestens eine lineare und gesättigte C₁₄-C₂₂-Alkanol allein aus Stearylalkohol ausgewählt ist.

9. Antitranspirant-Wachsstift gemäß Anspruch 1, 2, 3, 4, 5, 6, 7 oder 8, **dadurch gekennzeichnet, dass** der mindestens eine Öl-in-Wasser-Emulgator, der ein Ethoxylat von C₁₀-C₂₂-Alkanolen mit 8 bis 16 Ethylenoxid-Einheiten im Molekül darstellt, ausgewählt ist aus Mischungen aus Ceteth-12 und Steareth-12.

10. Antitranspirant-Wachsstift gemäß Anspruch 1, 2, 3, 4, 5, 6, 7, 8 oder 9, **dadurch gekennzeichnet, dass** der mindestens eine Öl-in-Wasser-Emulgator, der ein Ethoxylat von C₁₀-C₂₂-Alkanolen mit 8 bis 16 Ethylenoxid-Einheiten im Molekül darstellt, ausgewählt ist aus Mischungen aus Ceteth-12 und Steareth-12, wobei keine weiteren Öl-in-Wasser-Emulgatoren enthalten sind.

11. Antitranspirant-Wachsstift gemäß Anspruch 1, 2, 3, 4, 5, 6, 7, 8, 9 oder 10, **dadurch gekennzeichnet, dass** der Antitranspirant-Wirkstoff ausgewählt ist aus Aluminiumchlorhydrat und Aluminiumzirconiumchlorhydraten, insbesondere aus Aluminium-Zirconium-Tetrachlorohydrex-Glycin-Komplexen.

12. Antitranspirant-Wachsstift gemäß Anspruch 1, 2, 3, 4, 5, 6, 7, 8, 9, 10 oder 11, **dadurch gekennzeichnet, dass** das mindestens eine Wachs mit einem Schmelzpunkt von 25 bis < 50°C ausgewählt ist aus Mischungen aus Kokosfettsäureglycerinmono-, -di- und -triestern, Mischungen aus teilgehärteten oder vollständig gehärteten Kokosfettsäureglycerinmono-, -di-und -triestern, teilgehärteten oder vollständig gehärteten pflanzlichen Ölen, Butyrospermum Parkii (Shea Butter), Stearyllaurat, Cetearylstearat, Cetylpalmitat und Myristylmyristat sowie Mischungen hiervon.

13. Antitranspirant-Wachsstift gemäß Anspruch 1, 2, 3, 4, 5, 6, 7, 8, 9, 10, 11 oder 12, **dadurch gekennzeichnet, dass**, jeweils bezogen auf sein Gesamtgewicht, insgesamt 1 - 6 Gew.-%, bevorzugt 3 - 4 Gew.-%, mindestens eines Wachses mit einem Schmelzpunkt von 25 bis < 50°C enthalten ist.

14. Antitranspirant-Wachsstift gemäß Anspruch 1, 2, 3, 4, 5, 6, 7, 8, 9, 10, 11, 12 oder 13, **dadurch gekennzeichnet, dass** das mindestens eine nicht-flüchtige Öl ausgewählt ist aus Benzoesäure-C₁₂-C₁₅-Alkylester.

15. Antitranspirant-Wachsstift gemäß Anspruch 1, 2, 3, 4, 5, 6, 7, 8, 9, 10, 11, 12, 13 oder 14, **dadurch gekennzeichnet, dass**, jeweils bezogen auf sein Gesamtgewicht, insgesamt 1 - 4 Gew.-%, bevorzugt 1,5 - 2 Gew.-%, mindestens eines Wachses mit einem Schmelzpunkt von 65 - 150 °C enthalten ist, das besonders bevorzugt ausgewählt ist aus hydriertem Rizinusöl.

## Claims

1. An antiperspirant wax stick, containing
a) in total 3-27 wt.%, based on the total weight of the active substance (USP), which is free from water of crystallization, in the total composition, antiperspirant active ingredient(s),
b) 0-4 wt.% free water,
c) in total 30-70 wt.% of at least one cosmetic oil that is liquid under normal conditions, therein
ci) 10-50 wt.% of at least one volatile oil, which is selected from volatile cyclical silicones, volatile linear silicones and volatile aliphatic hydrocarbons, and mixtures thereof, and
cii) in addition to the at least one volatile oil, in total 1-30 wt.% of at least one non-volatile oil,
d) in total 15-25 wt.% of at least one wax component that is solid under normal conditions,
d)i. therein 12-22 wt.% of at least one linear and saturated C₁₄-C₂₂ alkanol,
d)ii. 0.5-5 wt.% of at least one wax having a melting point in the range of 65-150°C,
d)iii. 0.5-8 wt.% of at least one wax having a melting point in the range of 25 to < 50°C,
e) 0.5-6 wt.% of at least one water-insoluble particulate filler,
f) 1-4 wt.%, preferably 2-3.5 wt.%, particularly preferably 2.5-3 wt.%, of at least one oil-in-water emulsifier, selected from ethoxylates of C₁₀-C₂₂ alkanols having 8 to 16 ethylene oxide units in the molecule,
wherein, unless otherwise noted, all weight percentages are based on the total weight of the antiperspirant wax stick.

2. The antiperspirant wax stick according to claim 1, **characterized in that** the at least one volatile oil is contained in a total amount of from 15-40 wt.%, particularly preferably 20-35 wt.%, especially preferably 25-30 wt.%, in each case based on the total weight of the stick.

3. The antiperspirant wax stick according to one of claims 1 or 2, **characterized in that** at least one volatile oil is selected from cyclopentasiloxane.

4. The antiperspirant wax stick according to one of claims 1, 2 or 3, **characterized in that** at least one non-volatile oil is contained in a total amount of from 5-25 wt.%, preferably 8-20 wt.%, particularly preferably 10-15 wt.%, all weight percentages being based on the total weight of the antiperspirant wax stick.

5. The antiperspirant wax stick according to one of claims 1, 2, 3 or 4, **characterized in that** the at least one non-volatile oil has an index of refraction N_{D}²⁰ of at least 1.44, preferably from 1.445 to 1.54.

6. The antiperspirant wax stick according to one of claims 1, 2, 3, 4 or 5, **characterized in that** at least one non-volatile oil is selected from at least one addition product of 6 to 20, preferably 9 to 15 propylene oxide units of a monovalent or polyvalent C₃₋₂₂ alkanol, and is particularly preferably selected from PPG-14 butyl ether.

7. The antiperspirant wax stick according to one of claims 1, 2, 3, 4, 5 or 6, **characterized in that** the at least one non-volatile oil is contained, in each case based on the total weight of the stick, in a total amount of from 5-25 wt.%, preferably 8-20 wt.%, particularly preferably 10-15 wt.%.

8. The antiperspirant wax stick according to one of claims 1, 2, 3, 4, 5, 6 or 7, **characterized in that** at least one linear and saturated C₁₄-C₂₂ alkanol is selected from stearyl alcohol alone.

9. The antiperspirant wax stick according to one of claims 1, 2, 3, 4, 5, 6, 7 or 8, **characterized in that** the at least one oil-in-water emulsifier, which represents an ethoxylate of C₁₀-C₂₂ alkanols having 8 to 16 ethylene oxide units in the molecule, is selected from mixtures of Ceteth-12 and Steareth-12.

10. The antiperspirant wax stick according to one of claims 1, 2, 3, 4, 5, 6, 7, 8 or 9, **characterized in that** the at least one oil-in-water emulsifier, which represents an ethoxylate of C₁₀-C₂₂ alkanols having 8 to 16 ethylene oxide units in the molecule, is selected from mixtures of Ceteth-12 and Steareth-12, no additional oil-in-water emulsifiers being contained.

11. The antiperspirant wax stick according to one of claims 1, 2, 3, 4, 5, 6, 7, 8, 9 or 10, **characterized in that** the antiperspirant active ingredient is selected from aluminum chlorohydrate and aluminum zirconium chlorohydrates, in particular from aluminumzirconium tetrachlorohydrex glycine complexes.

12. The antiperspirant wax stick according to one of claims 1, 2, 3, 4, 5, 6, 7, 8, 9, 10 or 11, **characterized in that** the at least one wax having a melting point of from 25 to < 50°C is selected from mixtures of coconut fatty acid glycerol mono-, di- and triesters, mixtures of partially cured or completely cured coconut fatty acid glycerol mono-, di- and triesters, partially cured or completely cured plant oils, Butyrospermum parkii (shea butter), stearyl laurate, cetearyl stearate, cetyl palmitate and myristyl myristate and mixtures thereof.

13. The antiperspirant wax stick according to one of claims 1, 2, 3, 4, 5, 6, 7, 8, 9, 10, 11 or 12, **characterized in that**, in each case based on its total weight, in total 1-6 wt.%, preferably 3-4 wt.%, of at least one wax having a melting point of from 25 to < 50°C is contained.

14. The antiperspirant wax stick according to one of claims 1, 2, 3, 4, 5, 6, 7, 8, 9, 10, 11, 12 or 13, **characterized in that** the at least one non-volatile oil is selected from benzoic acid C₁₂-C₁₅ alkyl esters.

15. The antiperspirant wax stick according to one of claims 1, 2, 3, 4, 5, 6, 7, 8, 9, 10, 11, 12, 13 or 14, **characterized in that**, in each case based on its total weight, in total 1-4 wt.%, preferably 1.5-2 wt.%, of at least one wax having a melting point of from 65-150°C is contained, which is preferably selected from hydrogenated castor oil.

## Revendications

1. Stick antitranspirant contenant :
a) au total 3 à 27 % en poids d'agent(s) actif(s) antitranspirant(s), rapporté au poids total de la substance active sans eau de cristallisation (UPS) dans la composition totale,
b) 0 à 4 % en poids d'eau libre,
c) au total 30 à 70 % en poids d'au moins une huile cosmétique liquide en conditions normale, dont
ci) 10 à 50 % en poids d'au moins une huile liquide choisie parmi des silicones cycliques liquides, des silicones linéaires liquides, des hydrocarbures aliphatiques liquides, ainsi que leurs mélanges, et
cii) en plus de l'au moins une huile liquide, au total 1 à 30 % en poids d'au moins une huile non-liquide,
d) au total 15 à 25 % en poids d'au moins un composant cire solide en conditions normales, dont
d)i 12 à 22 % en poids d'au moins un C₁₄₋₂₂-alcool linéaire et saturé,
d)ii 0,5 à 5 % en poids d'au moins une cire avec un point de fusion entre 65 et 150°C,
d)iii 0,5 à 8 % en poids d'au moins une cire avec un point de fusion entre 25 et moins de 50°C,
e) 0,5 à 6 % en poids d'au moins une charge insoluble dans l'eau en forme de particules,
f) 1 à 4 % en poids, de préférence 2 à 3,5 % en poids, particulièrement préférentiellement 2,5 à 3 % d'au moins un émulsifiant huile dans l'eau choisi parmi des éthoxylates de C₁₀₋₂₂-alcools saturés, avec 8 à 16 unités oxyde d'éthylène dans la molécule,
où, sauf mention contraire, tous les pourcentages en poids se rapportent au poids total du stick antitranspirant.

2. Stick antitranspirant selon la revendication 1, **caractérisé en ce que** l'au moins une huile liquide est contenue à hauteur d'au total 15 à 40 % en poids, particulièrement préférentiellement 20 à 35 % en poids, extraordinairement préférentiellement 25 à 35 % en poids, respectivement rapporté au poids total du stick.

3. Stick antitranspirant selon la revendication 1 ou 2, **caractérisé en ce que** l'au moins une huile liquide choisie est le cyclopentasiloxane.

4. Stick antitranspirant selon la revendication 1, 2 ou 3, **caractérisé en ce que** l'au moins une huile non-liquide est contenue à hauteur d'au total 5 à 25 % en poids, de préférence 8 à 20 % en poids, particulièrement préférentiellement 10 à 15 % en poids, tous les pourcentages se rapportant respectivement au poids total du stick.

5. Stick antitranspirant selon la revendication 1, 2, 3 ou 4, **caractérisé en ce que** l'au moins une huile non-liquide présente un indice de réfraction n_{D}²⁰ d'au moins 1,44, de préférence de 1,445 à 1,54.

6. Stick antitranspirant selon la revendication 1, 2, 3, 4 ou 5, **caractérisé en ce que** l'au moins une huile non-liquide est choisie parmi au moins un produit de greffage de 6 à 20, de préférence de 9 à 15 unités oxyde de propylène sur un C₃₋₂₂-alcool monovalent ou polyvalent, particulièrement préférentiellement le PPG-14 butyléther.

7. Stick antitranspirant selon la revendication 1, 2, 3, 4, 5 ou 6, **caractérisé en ce que** l'au moins une huile non-liquide est contenue à hauteur d'au total 5 à 25 % en poids, de préférence 8 à 20 % en poids, particulièrement préférentiellement 10 à 15 % en poids rapporté au poids total du stick.

8. Stick antitranspirant selon la revendication 1, 2, 3, 4, 5, 6 ou 7, **caractérisé en ce que** l'au moins un C₁₄₋₂₂-alcool linéaire et saturé seul est choisi uniquement comme l'alcool stéarylique.

9. Stick antitranspirant selon la revendication 1, 2, 3, 4, 5, 6, 7 ou 8, **caractérisé en ce que** l'au moins un émulsifiant huile dans l'eau, qui représente un éthoxylate de C₁₀₋₂₂-alcools saturés avec 8 à 16 unités oxyde d'éthylène dans la molécule, est choisi parmi des mélanges de Ceteth 12 et de Steareth 12.

10. Stick antitranspirant selon la revendication 1, 2, 3, 4, 5, 6, 7, 8 ou 9, **caractérisé en ce que** l'au moins un émulsifiant huile dans l'eau, qui représente un éthoxylate de C₁₀₋₂₂-alcools saturés avec 8 à 16 unités oxyde d'éthylène dans la molécule, est choisi parmi des mélanges de Ceteth 12 et de Steareth 12, sans qu'il soit contenu aucun autre émulsifiant.

11. Stick antitranspirant selon la revendication 1, 2, 3, 4, 5, 6, 7, 8, 9 ou 10, **caractérisé en ce que** l'agent antitranspirant et choisi parmi le chlorhydrate d'aluminium et les chlorhydrates d'aluminium et de zirconium, en particulier les complexes aluminiumzirconium-tétrachlorohydrex-glycine.

12. Stick antitranspirant selon la revendication 1, 2, 3, 4, 5, 6, 7, 8, 9, 10 ou 11, **caractérisé en ce que** l'au moins une cire avec un point de fusion entre 25 et moins de 50°C est choisie parmi des mélanges de mono, di et triesters d'acide gras de coco et de glycérine, des mélanges de mono, di et triesters d'acide gras de coco et de glycérine partiellement ou totalement durcis, d'huiles végétales partiellement ou totalement durcies, de beurre de karité, de laurate de stéaryle, de stéarate de cétéaryle, de palmitate de cétyle et de myristate de myristyle, ainsi que leurs mélanges.

13. Stick antitranspirant selon la revendication 1, 2, 3, 4, 5, 6, 7, 8, 9, 10, 11 ou 12, **caractérisé en ce qu'**il contient au moins une cire avec un point de fusion entre 25 et moins de 50°C à hauteur d'au total 1 à 6 % en poids, de préférence 3 à 4 % en poids, rapporté à son poids total.

14. Stick antitranspirant selon la revendication 1, 2, 3, 4, 5, 6, 7, 8, 9, 10, 11, 12 ou 13, **caractérisé en ce que** l'huile non-liquide est choisie parmi les benzoates de C₁₂₋₁₅-alkyle.

15. Stick antitranspirant selon la revendication 1, 2, 3, 4, 5, 6, 7, 8, 9, 10, 11, 12, 13 ou 14, **caractérisé en ce qu'**il contient au moins une cire avec un point de fusion entre 65 à 150°C à hauteur d'au total 1 à 4 % en poids, de préférence 1,5 à 2 % en poids, rapporté à son poids total, qui est choisie particulièrement préférentiellement comme une huile de ricin hydrogénée.
